# EUROPEAN PATENT APPLICATION

(11) **EP 2 412 371 A1**
(43) Date of publication of application: **01.02.2012**
(21) Application number: 11003922.9
(22) Date of filing: 13.07.2007
(51) Int. Cl.: A61K 31/4196, A61K 31/4412, A61K 31/7048, A61P 31/10

(54) **Compositions comprising iron chelators (deferiprone/deferasirox) for use in the treatment of mucormycosis and other fungal diseases**

(30) Priority: 13.07.2006 US 831099 P; 26.01.2007 US 897620 P; 27.02.2007 US 904075 P
(62) Divisional of application: 07796874.1
(71) Applicant: Los Angeles Biomedical Research Institute at Harbor-UCLA Medical Center, Torrance, CA 90502-2064 (US)
(72) Inventor: Ibrahim, Asharf S., Torrance CA 90502 (US); Spellberg, Brad J., Rancho Palos Verdes CA 90275 (US); Edwards, John E., Palos Verdes CA 90274 (US)
(74) Representative: Müller-Boré & Partner Patentanwälte

(57) **Abstract**

The invention provides a composition including at least one iron chelating compound for use in a method of treating or preventing a fungal condition. The composition can include the iron chelating compounds deferiprone or deferasirox. An antifungal agent included in the composition can include a polyene antifungal agent, an azole antifungal agent or an echinocandin antifungal agent. The method includes administering to an individual having, or susceptible to having, a fungal condition a therapeutically effective amount of at least one iron chelating compound for a sufficient time to reduce the severity of a fungal condition, wherein the iron chelating compound comprises a non-siderophore or non-xenosiderophore relative to the fungal condition. Provided further by the invention is the prophylactic administration of the at least one iron chelating compound or at least one iron chelating compound prior to onset of the fungal condition.

SEQUENCE LISTING

<110> LOS ANGELES BIOMEDICAL RESEARCH INSTITUTE AT
      HARBOR-UCLA MEDICAL CENTER

<120> COMPOSITIONS AND METHODS FOR THE TREATMENT OF
      MUCORMYCOSIS AND OTHER FUNGAL DISEASES

<130> 066742-0053

<140> PCT/US07/16065
<141> 2007-07-13

<150> 60/904,075
<151> 2007-02-27

<150> 60/897,620
<151> 2007-01-26

<150> 60/831,099
<151> 2006-07-13

<160> 8

<170> PatentIn Ver. 3.3

<210> 1
<211> 19
<212> DNA
<213> Artificial Sequence

<220>
<223> Description of Artificial Sequence: Synthetic
      primer

<400> 1
tcagagaagg acttgaagc                                                19

<210> 2
<211> 19
<212> DNA
<213> Artificial Sequence

<220>
<223> Description of Artificial Sequence: Synthetic
      primer

<400> 2
taagtagccg tattgttcc                                                19

<210> 3
<211> 22
<212> DNA
<213> Artificial Sequence

<220>
<223> Description of Artificial Sequence: Synthetic
      primer

<400> 3
catggttgag attgtaagat ag                                            22

<210> 4
<211> 19
<212> DNA

<213> Artificial Sequence

<220>
<223> Description of Artificial Sequence: Synthetic
      primer

<400> 4
agtcaatgga cgtggagtc                                                    19


<210> 5
<211> 29
<212> DNA
<213> Artificial Sequence

<220>
<223> Description of Artificial Sequence: Synthetic
      primer

<400> 5
gctctagatc agtctcaaca ccatcaatt                                         29


<210> 6
<211> 29
<212> DNA
<213> Artificial Sequence

<220>
<223> Description of Artificial Sequence: Synthetic
      primer

<400> 6
tgcggccgtg ctttttagtt ctccttgga                                         29


<210> 7
<211> 26
<212> DNA
<213> Artificial Sequence

<220>
<223> Description of Artificial Sequence: Synthetic
      primer

<400> 7
gctctagatg gtattatcga tttaga                                            26


<210> 8
<211> 27
<212> DNA
<213> Artificial Sequence

<220>
<223> Description of Artificial Sequence: Synthetic
      primer

<400> 8
tacggccgca taccggaacc gttatcg                                           27

## Description

### DESCRIPTION OF THE INVENTION

### Field of the Invention

This invention relates generally to the treatment of infectious diseases and, more specifically to effective treatments of opportunistic fungal diseases.

### Background of the Invention

Mucormycosis is a life-threatening infection caused by fungi of the class Zygomycetes, order Mucorales. Fungi belonging to the order Mucorales are distributed into six families, all of which can cause mucormycosis (Ibrahim et al. Zygomycosis, p. 241-251, In W. E. Dismukes, P. G. Pappas, and J. D. Sobel (ed.), Clinical Mycology, Oxford University Press, New York (2003); Kwon-Chung, K. J., and J. E. Bennett, Mucormycosis, p. 524-559, Medical Mycology, Lea & Febiger, Philadelphia (1992), and Ribes et al. Zygomycetes in Human Disease, Clin Microbiol Rev 13:236-301 (2000)). However, fungi belonging to the family *Mucoraceae,* and specifically the species *Rhizopus oryzae (Rhizopus arrhizus),* are by far the most common cause of infection (Ribes et al., *supra).* Increasing cases of mucormycosis have been also reported due to infection with *Cunninghamella spp.* in the *Cunninghamellaceae* family (Cohen-Abbo et al., Clinical Infectious Diseases 17:173-77 (1993); Kontoyianis et al., Clinical Infectious Diseases 18:925-28 (1994); Kwon-Chung et al., American Journal of Clinical Pathology 64:544-48 (1975), and Ventura et al., Cancer 58:1534-36 (1986)). The remaining four families of the *Mucorales* order are less frequent causes of disease (Bearer et al., Journal of Clinical Microbiology 32:1823-24 (1994); Kamalam and Thambiah, Sabouraudia 18:19-20 (1980); Kemna et al., Journal of Clinical Microbiology 32:843-45 (1994); Lye et al., Pathology 28:364-65 (1996), and Ribes et al., *(supra)).*

The agents of mucormycosis are opportunistic pathogens that almost uniformly affect immunocompromised hosts (Spellberg et al., Clin. Microbiol. Rev. 18:556-69 (2005)). Patients in diabetic ketoacidosis are uniquely susceptible to mucormycosis, and develop these infections more commonly than infections caused by other fungi. In contrast, in the face of other immunocompromising conditions predisposing patients to developing mucormycosis, including neutropenia and corticosteroid therapy, mucormuycosis is less common than other opportunistic fungal infections, such as those caused by *Candida* and *Aspergillus spp.* Overall, a recent population-based study estimated the incidence of mucormycosis to be 1.7 cases per million people per year, which translates to approximately 500 cases per year in the United States (Rees et al., Clinical Infectious Diseases 27:1138-47 (1998)). Nevertheless, in patients at higher risk, such as those undergoing allogeneic bone marrow transplantation, the prevalence of mucormycosis has been described to be as high as 2-3% (Maertens et al Bone Marrow Transplantation 24:307-12 (1999); Marty et al., N Engl J Med 350:950-52 (2004)). Furthermore, a recent review found a marked increase in the incidence of mucormycosis over the past two decades (Gleissner et al., Leuk Lymphoma 45:1351-60 (2004)). Similar increases have been reported by major stem cell transplant centers. For example, reports have described a greater than doubling in the incidence of mucormycosis over the last two decades (Marr et al., Clin Infect Dis 34:909-17 (2002); Kontoyianis et al., Clin Infect Dis 30:851-56 (2000)). Given the increasing prevalence of diabetes, cancer, and organ transplantation in the aging United States population, the rise in incidence of mucormycosis is anticipated to continue unabated for the foreseeable future.

Available therapies for invasive mucormycosis include attempts to reverse the underlying predisposing factors, emergent, wide-spread surgical debridement of the infected area, and adjunctive antifungal therapy (Edwards, J., Jr., Zygomycosis, p. 1192-1199. In P. Hoeprich and M. Jordan (ed.), Infectious Disease, 4th ed. J.B. Lippincott Co., Philadelphia (1989); Ibrahim et al., (2003), *supra*; Kwon-Chung and Bennett, *supra;* Sugar, A. M., Agent of Mucormycosis and Related Species, p. 2311-2321. In G. Mandell, J. Bennett, and R. Dolin (ed.), Principles and Practices of Infectious Diseases, 4th ed. Churchill Livingstone, New York (1995)). Amphotericin B (AmB) remains the only antifungal agent approved for the treatment of invasive mucormycosis (*Id.*). Because the fungus is relatively resistant to AmB, high doses are required, which frequently cause nephrotoxicity and other adverse effects (Sugar, *supra*). Also, in the absence of surgical removal of the infected focus (such as excision of the eye in patients with rhinocerebral mucormycosis), antifungal therapy alone is rarely curative (Edwards, J. (1989), *supra*; Ibrahim et al., (2003), *supra*). Even when surgical debridement is combined with high-dose AmB, the mortality associated with mucormycosis exceeds 50% (Sugar, *supra*). In patients with disseminated disease mortality approaches 100% (Husain et al., Clin Infect Dis 37:221-29 (2003)). Because of this unacceptably high mortality rate, and the extreme morbidity of highly disfiguring surgical therapy, it has been imperative to develop new strategies to treat and prevent invasive mucormycosis.

The nephrotoxicity ofAmB has prompted clinicians in practice to adopt the use of lipid formulations of AmB, which are less nephrotoxic than AmB and can be administered at higher doses for a longer period of time (Ibrahim et al., (2003), *supra*). Several case reports of patients with mucormycosis have documented successful treatment with up to 15 mg/kg/d of lipid formulations of amphotericin (Cagatay et al., BMC Infect Dis 1:22 (2001); Ericsson et al., Clinical Infectious Diseases 16:585-56 (1993); Walsh et al., Antimicrob Agents Chemother 45:3487-96 (2001)). Nevertheless, the mortality of patients treated with lipid formulations of amphotericin remains high, underscoring the need for new therapeutic agents for this deadly disease.

Iron is required by virtually all microbial pathogens for growth and virulence (Howard, D. H., Clin Microbiol Rev 12:394-404 (1999)). In mammalian hosts, very little serum iron is available to microorganisms because it is highly bound to carrier proteins such as transferrin (Artis et al., Diabetes 31:1109-14. (1982)). Sequestration of serum iron is a major host defense mechanism against R. oryzae in particular (Artis et al., *supra*). The organism grows poorly in serum and this growth inhibition is reversed when exogenous iron is added (Artis et al., *supra*; Boelaert et al., Journal of Clinical Investigation 91:1979-86 (1993)).

Patients with elevated levels of available serum iron are uniquely susceptible to infection by *R. oryzae* and other Zygomycetes, but to a lesser extent to other pathogenic fungi, such as *Candida or Aspergillus* (Ibrahim et al., (2003), *supra*; Sugar, *supra*). For example, patients treated with the iron chelator, deferoxamine, have a markedly increased incidence of invasive mucormycosis, which is associated with a mortality of >80% in these patients (Boelaert et al., Kidney International 45:667-71 (1994)). While deferoxamine acts as an iron chelator with respect to the human host, its effect on *R. oryzae* appears to be just the opposite. Deferoxamine predisposes patients to *Rhizopus* infection by acting as a siderophore, which supplies previously unavailable iron to the fungus (Boelaert et al., (1993), *supra*). *Rhizopus* obtains iron from the iron-deferoxamine complex by intracellular transport of the reduced iron without deferoxamine internalization (de Locht et al., Biochemical Pharmacology 47:1843-50 (1994)). This transport is likely mediated by high-affinity iron permeases. Therefore, increased available serum iron is a risk factor for mucormycosis pathogenesis. However, iron chelating compounds can function as sideophores for the pathogen and, thus, may not be generally applicable for therapeutic treatments.

Thus, there exists a need for compounds and methods that can reduce the risk of mucormycosis pathogenesis and provide effective therapeutic treatment. The present invention satisfies this need and provides related advantages as well.

### SUMMARY OF THE INVENTION

The invention provides a composition including at least one iron chelating compound and at least one antifungal agent. The composition can include the iron chelating compounds deferiprone or deferasirox. An antifungal agent included in the composition can include a polyene antifungal agent, an azole antifungal agent or an echinocandin antifungal agent. The invention also provides a method of treating or preventing a fungal condition. The method includes administering to an individual having, or susceptible to having, a fungal condition a therapeutically effective amount of at least one iron chelating compound for a sufficient time to reduce the severity of a fungal condition, wherein the iron chelating compound comprises a non-siderophore or non-xenosiderophore relative to the fungal condition. A method of treating or preventing a fungal condition provided by the invention also can include administering to an individual having, or susceptible of having, a fungal condition a therapeutically effective amount of at least one iron chelating compound and at least one antifungal agent. Provided further by the invention is a method including prophylactic administration of the at least one iron chelating compound or at least one iron chelating compound and at least one antifungal agent prior to onset of the fungal condition.

### BRIEF DESCRIPTION OF DRAWINGS

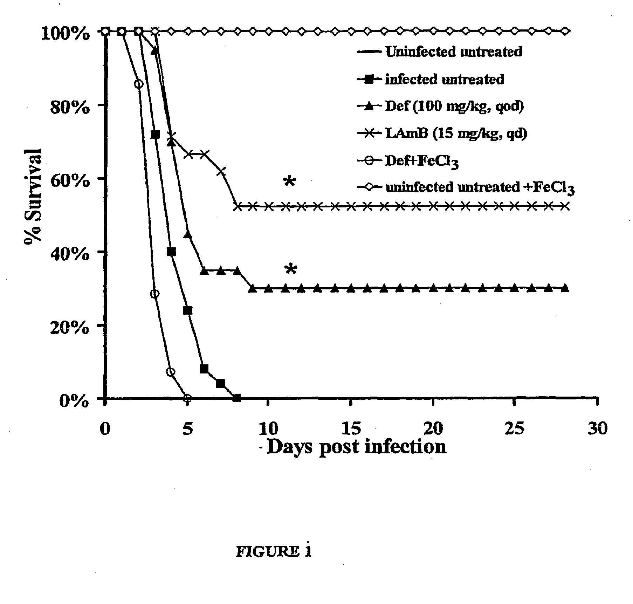

Figure 1 shows the relationship between Def and iron and survival ofDKA mice infected with *R. oryzae.* Mice (n≥20 in each treatment) were treated with deferiprone (Def) or Def + FeCl₃ (60 mg/kg) to reverse the effect of iron chelation. An LAmB-treated arm was included as a control. Treatment was initiated 24 h post infection. *, p<0.003 compared to infected untreated or uninfected untreated +FeCl₃.

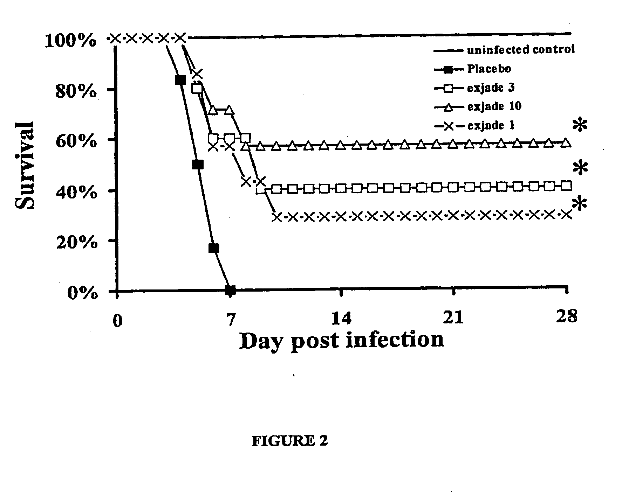

Figure 2 shows that Exjade^{Tm} (deferasirox) improves survival of diabetic ketoacidotic mice infected with *R. oryzae.* Mice (n=6 for placebo and 7 for Exjade®) were infected through tail vein injection with 2.2 x 10⁴ spores of *R. oryzae* 99-892 (clinical isolate obtained from the lung of infected patient). Twenty four hours post infection mice were treated by oral gavage with placebo, 1,3, or 10 mg/kg of Exjade® bid for 7 consecutive days. *, p<0.05 compared to placebo.

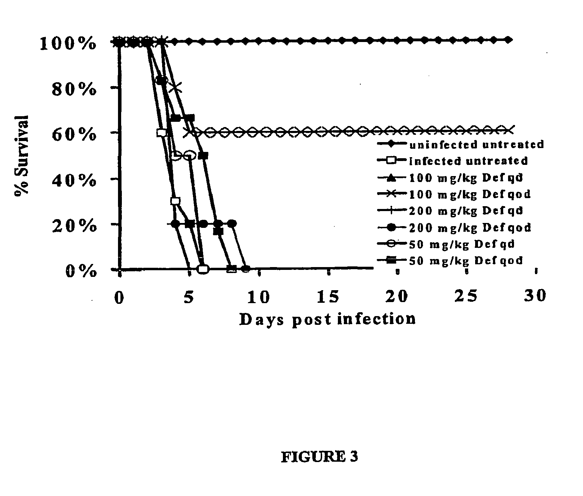

Figure 3 shows the survival ofDKA mice infected with R. oryzae and treated with different treatment regimens of Def. Mice (n=6 per group) were treated 24 h post infection with Def at 50, 100, or 200 mg/kg every day (qd) or every other day (qod) for a total of 4 doses. *, p<0.05 compared to infected untreated.

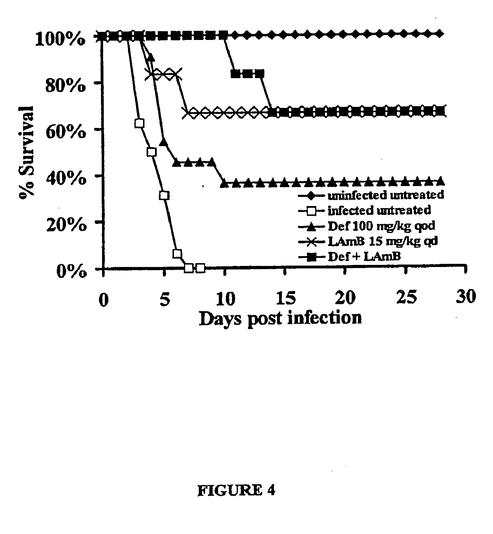

Figure 4 shows the treatment of experimental mucormycosis with a combination of Def and LAmB. Mice (n=11, for infected untreated and Def and n=6 LAmB and Def+ LAmB) were infected with *R. oryzae* then treated 24 h later with Def, LAmB, or a combination of both drugs for a total of 4 doses. *, p<0.005 compared to infected untreated mice.

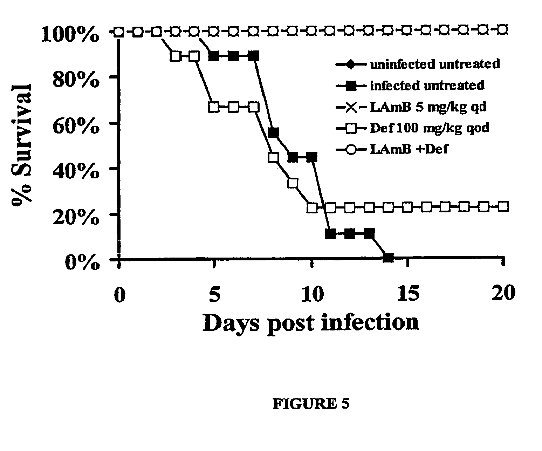

Figure 5 shows the survival of neutropenic mice infected with *R. oryzae* and prophylaxed with Def, LAmB, or both. Mice (n=9) were treated on day -1 with Def qod, LAmB qd, or both for a total of four doses. *p < 0.05 for LAmB or LAmB + Def vs. infected untreated.

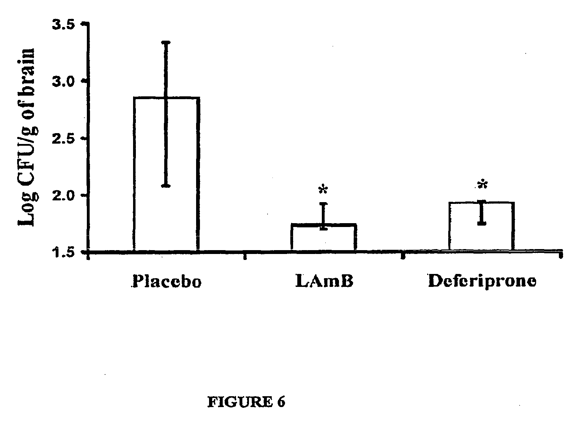

Figure 6 shows the brain *R. oryzae* burden of DKA mice (n=6) treated with deferiprone, LAmB or placebo. Mice were infected with 3.8 x 103 spores and brains harvested 54 h later after two doses of treatment with either drug. Data are displayed as medians + interquartile ranges. The y axis reflects the lower limit of detection of the assay. *, *P*< 0.036 vs. placebo by Steel test for multiple comparisons.

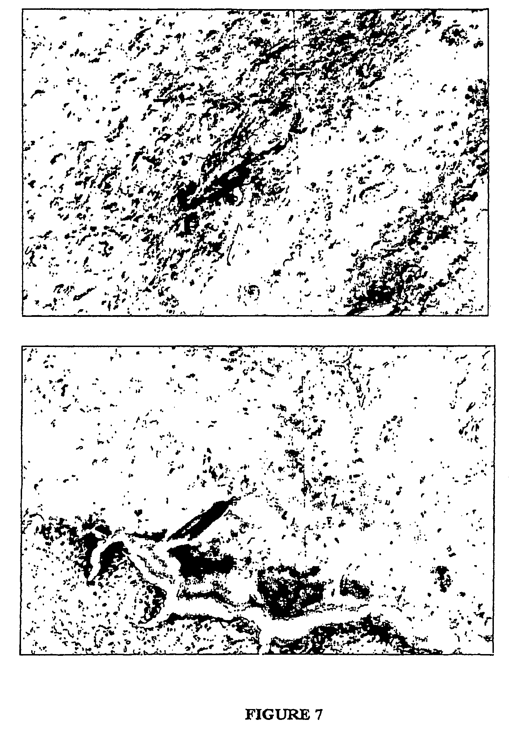

Figure 7 shows a frozen section ofnecrotic nasal mucosa stained with silver methanamine, showing fungi with wide, ribbon-like aseptate hyphae consistent with Mucorales. 840x magnification.

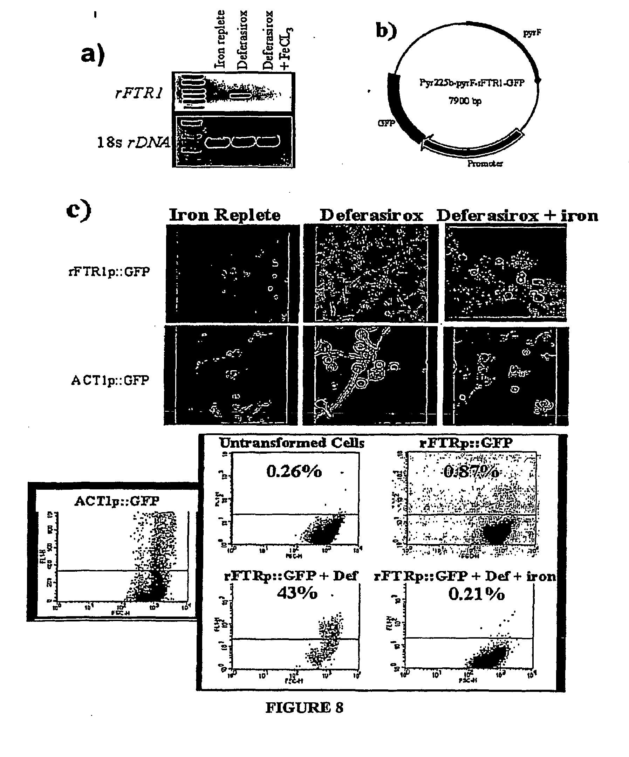

Figure 8 shows that deferasirox induces *rFTR1* gene expression in *Rhizopus oryzae.* Specifically, Figure 8(a) shows the RT PCR-detected expression of *rFTR1* gene from *R. oryzae* Mycelia incubated in iron-replete, iron chelation (deferasirox), or reversal of iron chelation (deferasirox saturated with ferric chloride) conditions. The expression of 18s rDNA was included to verify the quality of RNA extraction. Figure 8(b) shows a diagram demonstrating the strategy for constructing *R. oryzae* GFP expression vector. Promoter denotes either rFtr1p or Actlp. Figure 8(c) shows the GFP expression in *R. oryzae* driven by rFtr1p or Act1p (as determined by confocal microscopy and flow cytometry) of *R. oryzae* grown in iron-replete, deferasirox, or deferasirox saturated with ferric chloride-containing media. GFP expression is revealed by green fluorescent cells by confocal microscopy, and percent of fluorescent cells in channel FL1 (y axis) by flow cytometry.

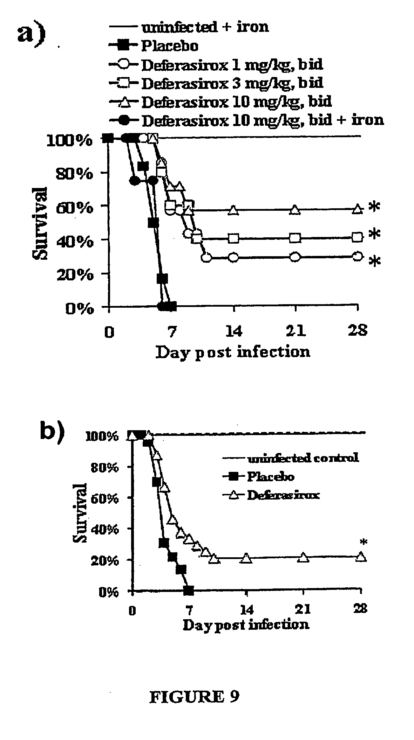

Figure 9(a) shows the survival of diabetic ketoacidotic mice (n> 7 per group) infected with *R. oryzae* 99-892 (2.2 x 10⁴) and treated with different doses of deferasirox. Mice were treated with placebo (hydroxypropylcellulose carrier), deferasirox, or deferasirox plus iron (FeCl₃, 10 mg/kg) to reverse the effects of iron chelation. **P* < 0.05 for survival. Figure 9(b) shows the survival of diabetic ketoacidotic mice (n=24 from three separate experiments with similar results) infected with *R. oryzae* 99-880 (average inoculum 1.3 x 10³ spores) and 24 h later treated with 10 mg/kg deferasirox bid for 7days. **P* <0.003 compared to placebo.

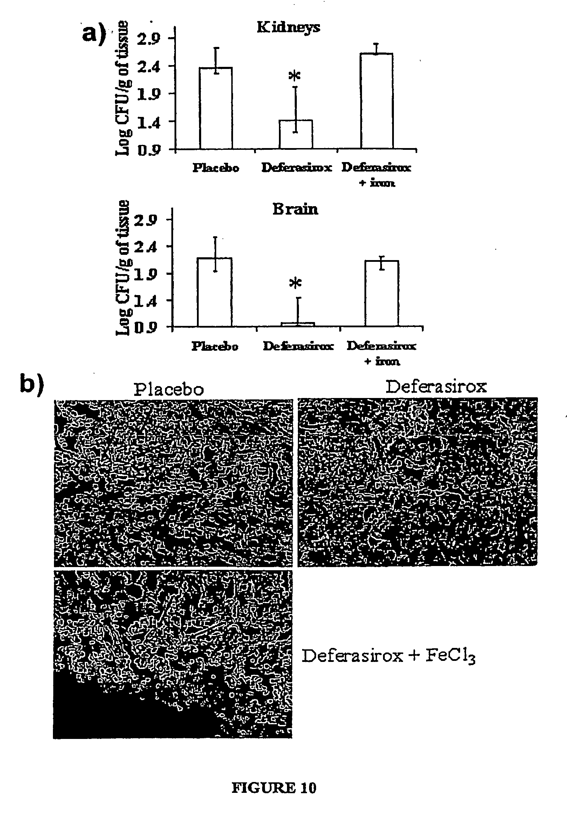

Figure 10(a) shows the brain and kidney fungal burden of diabetic ketoacidotic mice (n=11 I per group) infected with *R. oryzae* 99-892 (4.2 x 10⁴ spores) and treated with placebo, deferasirox (10 mg/kg bid), or deferasirox plus iron. Organs were harvested on day 4 after receiving three daily treatments. Data are displayed as median ± interquartile ranges. The y-axis reflects the lower limit of detection of the assay. Figure 10(b) shows hematoxylin and eosin stained kidney sections of diabetic ketoacidotic mice infected with *R. oryzae* 99-892 and treated with deferasirox, deferasirox plus ferric chloride, or placebo as mentioned in 10(a). Arrows indicate *R. oryzae* hyphae in the tissue. Magnification, X 400. **P* <0.002 for tissue fungal burden compared to placebo or deferasirox plus ferric chloride.

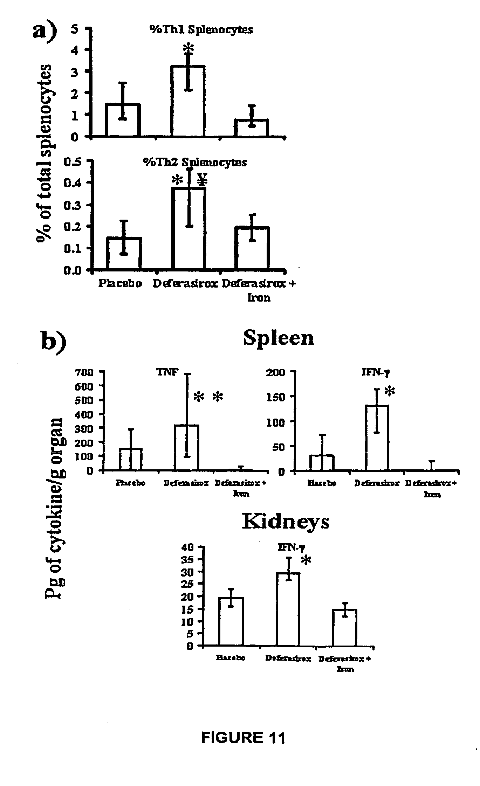

Figure 11 shows that Iron chelation increases splenic Th1 and Th2 lymphocyte frequencies and increases the levels of pro-inflammatory cytokines compared to iron overloaded mice. In particular, Figure 11 (a) shows the splenic Th1 and Th2 lymphocyte frequencies in diabetic ketoacidotic mice (n=11) infected with 3.1 x 10⁴ spores of *R. oryzae* 99-892 and, 24 h later, treated with placebo, deferasirox, or deferasirox plus ferric chloride. Figure 11(b) shows the results of whole organ cytokine analysis by Cytometric Bead Array^{™} of the same mice (n=11), mentioned in 11(a), sacrificed and spleens and kidneys collected 4 days post infection. Data in both 11 (a) and (b) are displayed as median ± interquartile ranges. **P* <0.02 compared to placebo or deferasirox plus ferric chloride. ***P* <0.05 compared and ¥ *P* <0.07 compared to deferasirox plus ferric chloride.

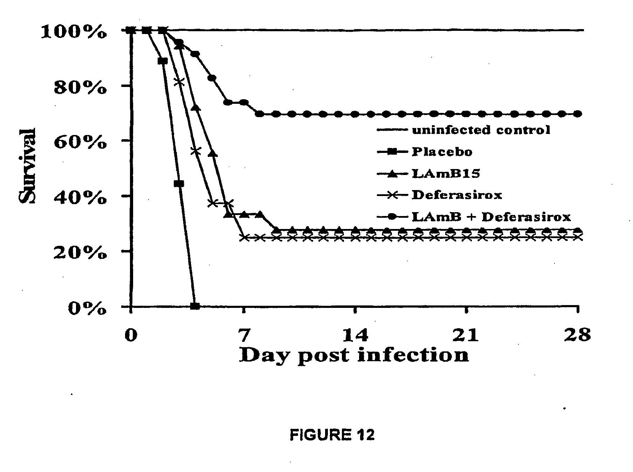

Figure 12 shows the efficacy against mucormycosis for deferasirox alone, LAmB alone and the combination of deferasirox and LAmB together. The figure specifically shows the survival of diabetic ketoacidotic mice (n > 16 from two separate experiments with similar results) infected with *R. oryzae* 99-880 (average inoculum 1.5 x 10³ spores) and treated with deferasirox alone, LAmB alone and the combination of deferasirox (10 mg/kg bid for 7 days) and LAmB (15 mg/kg for 4 days).

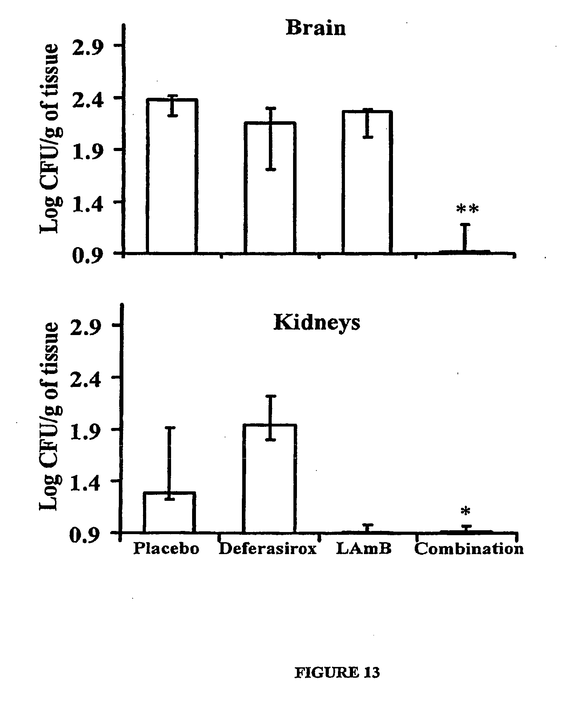

Figure 13 shows the efficacy in reducing target organ infection for deferasirox alone, LAmB alone and the combination of deferasirox and LAmB together. More specifically, the figure shows tissue *R. oryzae* burden in brains and kidneys of mice (n > 7) infected with *R oryzae* 99-880. In these mice, treatment began 24 h post infection and consisted of placebo, deferasirox (10 mg/kg, bid), LAmB (15 mg/kg/d), or a combination of both drugs. Organs were harvested on day 3 after receiving two daily treatments. Data are displayed as median ± interquartile ranges. The y-axis reflects the lower limit of detection of the assay. **P* <0.003 compared to placebo. ***P* <0.003 compared to placebo, deferasirox , or LAmB. ¥ *P* <0.01 compared to placebo, or deferasirox.

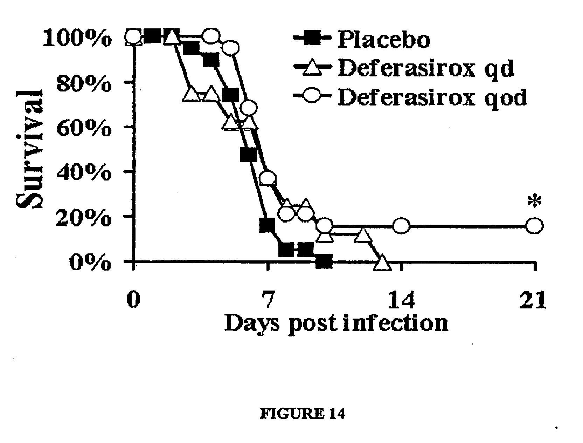

Figure 14 shows the efficacy of deferasirox in treating *R. oryzae* infections in neutropenic mice. Cyclophosphamide-treated mice (n=19 from two separate experiments with similar results) were infected with 2.7 x 10³ spores of *R. oryzae* 99-892. Mice were treated 24 h pot infection with placebo, or deferasirox (10 mg/kg) administered every day (qd) or every other day (qod) for a total of 5 doses. **P* =0.037 compared to placebo.

### DETAILED DESCRIPTION OF THE INVENTION

This invention is directed to the use of iron chelating compounds for the treatment or reduction in severity of fungal conditions. The iron chelating compounds are selected to have low siderophore or xenosiderophore activity relative to the targeted fungal condition. In some embodiments, the iron chelating compounds are selected to be substantially free of siderophore or xenosiderophore activity relative to the targeted fungal condition. In contrast iron chelators that act as siderophores or xenosiderophores, which supply previously unavailable iron to a fungus, non-siderophoric and non-xenosiderophoric iron chelating compounds lack such facilitating or transport activity. Therefore, the iron chelators of the inventions can be used for removal of iron from the surrounding environment. One benefit of iron chelation for antifungal therapy is that it reduces the availability of a important mineral which is required by many microbial pathogens for growth and/or virulence.

In one embodiment, the invention is directed to a therapeutic composition containing a iron chelating compound and an antifungal agent. The iron chelating compound can be deferiprone (1,2 dimethyl-3-hydroxypyrid-4-1), or deferasirox (4-[3,5-Bis(2-hydroxyphenyl)-1H-1,2,4-triazol-1-yl]-benzoic acid). Both deferiprone and deferasirox have been used in therapeutic settings for the treatment of iron overload conditions and are therefore, safe and effective for iron chelation therapy for the treatment or prevention of fungal conditions. Antifungal agents known in the art can be selected for combination with an iron chelator of the invention. The combination is beneficial for use in the treatment or prevention of fungal conditions.

In another embodiment, the invention is directed to a method of treating or preventing a fungal condition. The method includes administering to an individual one or more iron chelating compounds which exhibits non-siderophoric or non-xenosiderophoric iron chelation activity toward the targeted fungal species. The method also can include co-administration of an antifungal agent to achieve enhanced efficacy compared to an iron chelator alone. The iron chelating compound can be deferiprone, deferasirox or selected from other iron chelating compounds know in the art. The methods ofthe invention are particularly beneficial for therapeutic and prophylactic treatments because iron chelation targets the removal of an important mineral for fungal pathogenesis.

The invention provides a composition including at least one iron chelating compound and at least one antifungal agent.

Iron is required by most fungal systems for growth, viability and/or virulence. Fungi have developed a variety of mechanisms for acquisition, uptake and methods of storage to ensure sufficient supplies ofthis important metal. The compositions ofthe invention target this important mineral for removal from the host environment to neutralize fungal pathogens. The compositions of the invention include an iron chelating compound to deplete available iron and inhibit growth, viability and/or virulence to a fungal pathogen.

The compositions of the invention include a combination of at least one iron chelating compound together with at least one antifungal agent. The inclusion of both an iron chelating compound and an antifungal agent combines antifungal activities that target two different pathways used by fungi for growth, viability or virulence. Targeting two or more different fungal pathways provides for effective therapeutic treatment of fungal conditions since the likelihood of pathogenic evasion of both targeted pathways is low.

As used herein, the term "iron chelating compound" or "iron chelator" is intended to mean a compound that binds iron between two or more separate binding sites so as to form a chelate ring or rings. An iron chelating compound bound or complexed with iron is referred to herein as an iron chelate. An iron chelating compound can be bidentate (or didentate), which binds iron using two separate binding sites. Iron chelating compounds of the invention also can be tridentate, tetradentate or higher order multidentate iron chelation compounds binding iron with three, four or more separate binding sites, respectively. Iron chelating compounds of the invention include chelation compounds that can bind to all oxidation states of iron including, for example, iron (-II) state, iron (-I) state, iron (0) state, iron (I) state, iron (II) state (ferrous), iron (III) state (ferric), iron (IV) state (ferryl) and/or iron (V). Iron chelation therapy refers to the use of an iron chelator to bind with iron *in vivo* to form an iron chelate so that the iron loses its toxic effect or adverse physiological activity. Alternatively, chelated iron becomes unavailable to the infectious organism.

An iron chelating compound useful in a composition of the invention can include any chelator or other molecule that can bind and prevent iron utilization by the targeted fungus or fungi. Specific examples of iron chelating compounds included in the compositions of the invention include, for example, deferiprone and deferasirox. These exemplary iron chelating compounds are particularly useful because they have been approved in various countries for therapeutic indications unrelated to fungal conditions and are therefore, well characterized, safe and non-toxic in humans.

The term "deferiprone," as it is used herein is intended to mean an iron chelating compound having the structure 1,2 dimethyl-3-hydroxypyrid-4-1. Deferiprone (Def), also is known in the art as L1, CP20, Ferriprox, or Kelfer. Deferiprone, is a member of the α-ketohydroxypyridine class of iron chelators and is commercially available from, for example, Apotex, Inc. (Weston, Ontario, Canada).

The term "deferasirox" as it is used herein is intended to mean an iron chelating compound having the structure 4-[3,5-Bis(2-hydroxyphenyl)-1H-1,2,4-triazol-1-yl]-benzoic acid and having a molecular weight of 373.4 daltons. Deferasirox, also is known in the art as Exjade® or ICL 670, is a member of the class of tridentate iron chelators referred to as N-substituted bis-hydroxyphenyl-triazoles. Deferasirox is commercially available from, for example, Novartis, Corp. (Basel, Switzerland), for example, under the trademark Exjade®. According to the present invention, the terms "deferasirox", "ICL670", "Exjade®" are meant to refer to the active ingredient 4-[3,5-Bis(2-hydroxyphenyl)-1H-1,2,4-triazol-1-yl]-benzoic acid, e.g. 4-[3,5-Bis(2-hydroxyphenyl)-1H-1,2,4-triazol-1-yl]-benzoic acid or a pharmaceutically acceptable salt thereof. Deferasirox, its process of manufacture and its uses are described in, for example, U.S. Patent Nos. 6,465,504B1 and 6,595,750 B2, and in European Patent No. EP0914118. Pharmaceutical preparations comprising 4-[3,5-Bis(2-hydroxyphenyl)-1H-1,2,4-triazol-1-yl]-benzoic acid or a pharmaceutically acceptable salt thereof are described in, for example, International Patent Application WO2004/035026.

Other iron chelating compounds also can be included in the compositions of the invention. Such other iron chelating compounds are well known in the art and include, for example, naturally occurring siderophores and xenosiderophores such as those described below as well and non-naturally occurring compounds such as deferiprone and deferasirox.

Non-naturally occurring iron chelating compounds are exemplified by members of the hydroxypyridin-4-one (HPO) class of chelators such as deferiprone, members of the N-substituted bis-hydroxyphenyl-triazole class of chelators such as deferasirox, diethylenetriaminepentaacetic acid (DTPA) and deferoxamine. Deferiprone, deferasirox and any of the above exemplary iron chelating compounds as well as others well known in the art can be included in the iron chelating compound containing compositions of the invention

Siderophores and xenosiderophores include, for example, hydroxamates and polycarboxylates. The hydroxamates contain an N-δ-hydroxyornithine moiety and are generally categorized into four exemplary families. One category includes rhodotorulic acid, which is the diketopiperazine of N-δ-acetyl-L-N δ-hydroxyornithine. Included within this category are derivatives such as dihydroxamate named dimerum acid. A second category includes the coprogens, which contain an N-δ-acyl-N-δ-hydroxy-L-ornithine moiety. Coprogens also can be considered trihydroxamate derivatives of rhodotorulic acid with a linear structure. A third category includes the ferrichromes, which consist of cyclic peptides containing a tripeptide of N-δ-acyl-N-δ-hydroxyornithine and combinations of glycine, serine or alanine. The fourth exemplary category includes the fusarinines, also called fusigens, which can be either linear or cyclic hydroxamates. Fusarinine is a compound characterized by N acylation of N-hydroxyornithine by anhydromevalonic acid.

The polycarboxylates consist of a citric acid-containing polycarboxylate called rhizoferrin. The molecule contains two citric acid units linked to diaminobutane. Rhizoferrin is widely distributed among the members of the phylum Zygomycota, having been observed in the order Mucorales and in the order Entomophthorales. Other categories of siderophores useful as iron chelating compounds in the compositions of the invention include, for example, the phenolate-catecholate class of siderophores, hernin, and β-ketoaldehyde phytotoxins.

The amount of iron chelating compound included in a composition of the invention can vary but will generally be a therapeutically effective amount or an amount that can be reconstituted or diluted to a therapeutically effective amount. For example, effective amounts of iron chelating compounds of the invention are described further below with reference to the methods of the invention. An amount of one, some or all iron chelating compounds can be formulated in a composition of the invention to correspond to these exemplary effective amounts.

An iron chelating compound also can be formulated in a composition of the invention in amounts greater than a therapeutically effective amount for either short or long-term storage and the end user can dilute the formulation prior to use to a desired therapeutically effective amount. Alternatively, an iron chelating compound included in a composition of the invention can be lyophilized or produced in powder or other solid form and the end user can reconstitute the dry formulation prior to use to a desired therapeutically effective amount.

The dry or concentrated formulations or the formulations containing an effective amount of constituents can contain the iron chelating compound and the antifungal agent alone or together with any desired excipients, surfactants, tonicifiers, salts or buffers. Dilution or reconstitution can be performed in a pharmaceutically acceptable medium that adjusts the formulation to the desired therapeutically effective amount of the at least one iron chelating compound and the at least one antifungal agent and includes any includes any additional excipients, surfactants, tonicifiers, salts or buffers. Pharmaceutical formulations are well known and practiced in the pharmaceutical. Any such well known formulations and pharmaceutical formulation components are applicable for use with a composition of the invention. Pharmaceutical formulations, excipients, their use, formulation and characteristics are well known in the art and can be found described in, for example, *Remington: The Science and Practice of Pharmacy, supra;* Williams et al., Foye's Principles of Medicinal Chemistry, 5th Ed., Lippincott Williams & Wilkins (2002); Allen et al., Ansels Pharmaceutical Dosage Forms and Drug Delivery Systems, 8th Ed., Lippincott Williams & Wilkins (2004). Similarly, surfactant, their use, formulation and characteristics are well known in the art and can be found described in, for example, Holmberg et al., *Surfactants and Polymers in Aqueous Solution, supra;* Surfactants: A Practical Handbook, K. Robert Lange, ed., *supra,* and Vogel, A.I., Vogel's Textbook of Practical Organic Chemistry*, supra.*

The compositions of the invention also include at least one antifungal agent. The term "antifungal agent" or "antifungal," as it is used herein is intended to mean an agent that destroys fungi, or inhibits or prevents fungal growth, viability and/or virulence. Exemplary categories of antifungal agents include polyene antifungal agents, azole antifungal agents and echinocandin antifungal agents. Specific examples of polyene antifungal agents include amphotericin B deoxycholate, liposomal amphotericin B, amphotericin B lipid complex and amphotec. Specific examples of azole antifungal agents include posaconazole, voricoazole, fluconazole and itraconazole. Specific examples of echinocandin antifungal agents include caspofungin acetate and micafungin. Numerous other antifungal agents are well known in the art and are included within the meaning of the term as it is used herein.

The combination of the at least one iron chelating compound and the at least one antifungal agent will be selected depending on the fungal condition to be targeted. For example, amphotericin B lipid complex can be a good antifungal agent against, for example, zygomycosis, (mucormycosis.), aspergillosis and/or candidiasis and can be combined with an iron chelating compound such as deferiprone or deferasirox. Similarly, one of the antifungal agents exemplified above or another one known in the art can be effective or therapeutically desirable against another targeted condition and one of such other antifungal agents can be combined with an iron chelating compound to produce a composition of the invention. Therefore, the compositions of the invention are flexible in both their constituent iron chelating compounds and constituent antifungal agents and allow for any an all combinations and permutations ofthe at least one iron chelating compound and the at least one antifungal agent of be combined, for example, into a single formulation.

Accordingly, in one embodiment, the invention provides for a composition containing at least one iron chelating compound and at least one antifungal agent. The iron chelating compound can be selected from, for example, the non-naturally occurring iron chelating compounds described previously exemplified by members of the hydroxypyridin-4-one (HPO) class of chelators such as deferiprone, members of the N-substituted bis-hydroxyphenyl-triazole class of chelators such as deferasirox, diethylenetriaminepentaacetic acid (DTPA) and deferoxamine. The iron chelating compound also can be selected from, for example, the siderophores and/or xenosiderophores exemplified by, for example, the hydroxamates, the polycarboxylates, the phenolate-catecholate class of siderophores, hemin or the β-ketoaldehyde phytotoxins described previously.

The antifungal agent can be selected from, for example, a polyene antifungal agent such as amphotericin B deoxycholate, liposomal amphotericin B, amphotericin B lipid complex or amphotec. The antifungal agent also can be selected from, for example, an azole antifungal agent such as posaconazole, voricoazole, fluconazole or itraconazole. The antifungal agent can additionally be selected from, for example, an echinocandin antifungal agent such as caspofungin acetate or micafungin. An exemplary composition of the invention having one iron chelating compound for the at least one iron chelating compound and having one antifungal agent for the at least one antifungal agent can be deferasirox and amphotericion B lipid complex.

As with the amount of an iron chelating compound included within a composition of the invention, the amount of antifungal agent included in a also can vary, but will generally be a therapeutically effective amount or an amount that can be reconstituted or diluted to a therapeutically effective amount. For example, effective amounts of antifungal agents of the invention are described further below and exemplified with reference to polyene antifungal agents. An amount of one, some or all antifungal agents can be formulated in a composition of the invention to correspond to these exemplary effective amounts for polyene antifungal agents or corresponding to well known effective amounts for other antifungal agents such as the azole antifungal agents or the echinocandin antifiangal agent. Similarly, and as described previously with respect the an iron chelating compound included in a composition of the invention, an antifungal agent also can be formulated in a composition of the invention in concentrated form for either short or long-term storage and the end user can dilute the formulation prior to use to a desired therapeutically effective amount. Additionally, an antifungal agent included in a composition of the invention can be produced in lyophilized, powder or other solid form and the end user can reconstitute the dry formulation prior to use to a desired therapeutically effective amount.

The dry or concentrated formulations or the formulations containing an effective amount of constituents can contain the iron chelating compound and the antifungal agent alone or together with any desired excipients, surfactants, tonicifiers, salts or buffers. Dilution or reconstitution can be performed as described previously and exemplified in, for example, Remington: The Science and Practice of Pharmacy, supra; Williams et al., Foye's Principles of Medicinal Chemistry, 5th Ed., Lippincott Williams & Wilkins (2002); Allen et al., Ansels Pharmaceutical Dosage Forms and Drug Delivery Systems, 8th Ed., Lippincott Williams & Wilkins (2004); Holmberg et al., Surfactants and Polymers in Aqueous Solution, supra; Surfactants: A Practical Handbook, K. Robert Lange, ed., supra, and Vogel, A.I., Vogel's Textbook of Practical Organic Chemistry, supra.

The compositions of the invention can additionally contain two or more iron chelating compounds. Inclusion of two or more iron chelating compounds allows targeting of multiple fungal conditions and/or provides for the inclusion of a range of iron affinities in the iron chelating component of the composition. Inclusion of iron chelating compounds having different affinities for iron can be therapeutically beneficial to further prevent evasion of fungal pathogens.

The compositions of the invention will generally contain between about 1-8, particularly between about 2-7, more particularly between about 3-6 or even more particularly between about 4-5 iron chelating compounds. Iron chelating compounds greater than or in between these ranges also can be used in a composition of the invention. For example, a composition of the invention can be produced that contains essentially any desired number of different iron chelating compounds including, for example, 1, 2, 3, 4, 5, 6, 7, 8, 9, 10, 11 or 12 or more.

The compositions of the invention can additionally contain two or more antifungal agents. As with the inclusion of two or more iron chelating compounds, the inclusion of two or more antifungal agents also allows targeting of multiple fungal conditions and/or provides for the targeting of different fungal mechanisms used for growth, viability or virulence. Inclusion of multiple antifungal agents can similarly be therapeutically beneficial to further prevent evasion of fungal pathogens.

Similarly, the compositions of the invention will generally contain between about 1-8, particularly between about 2-7, more particularly between about 3-6 or even more particularly between about 4-5 antifungal agents. Antifungal agents greater than or in between these ranges also can be used in a composition of the invention. Therefore, a composition of the invention can be produced that contains essentially any desired number of different antifungal agents including, for example, 1, 2, 3, 4, 5, 6, 7, 8, 9, 10, 11 or 12 or more.

Exemplary compositions include deferasirox and one or more of any of amphotericin B deoxycholate, liposomal amphotericin B, amphotericin B lipid complex, amphotec, posaconazole, voricoazole, fluconazole, itraconazole, caspofungin acetate or micafungin. Deferasirox and deferiprone and one or more of any of amphotericin B deoxycholate, liposomal amphotericin B, amphotericin B lipid complex, amphotec, posaconazole, voricoazole, fluconazole, itraconazole, caspofungin acetate or micafungin. Particularly useful compositions include, for example, deferasirox and/or deferiprone and one, two or three or more antifungal agents selected from each category corresponding to the polyene antifungal agents, the azole antifungal agents and the echinocandin antifungal agents. Therefore, the invention provides any combination of 1, 2, 3, 4, 5, 6, 7, 8, 9, 10, 11 or 12 or more iron chelating compounds together with any combination of 1, 2, 3, 4, 5, 6, 7, 8, 9, 10, 11 or 12 or more antifungal agents. Such compositions containing multiple iron chelating compounds and/or multiple antifungal agents can be formulated as described previously.

The compositions of the invention also can include a pharmaceutically acceptable medium. As used herein, the term "pharmaceutically acceptable medium" is intended to mean that the medium admixed with an iron chelating compound ofthe invention is ofsufficient purity and quality for use in humans. A pharmaceutically acceptable medium includes a formulation that is substantially free from contaminating particles and organisms. Therefore, the term is intended to include a medium that is compatible with a iron chelating compound of the invention and is safe and non-toxic when administered to humans. Such pharmaceutically acceptable media are well known in the art.

The invention also provides a method of treating or preventing a fungal condition. The method includes administering to an individual having, or susceptible to having, a fungal condition a therapeutically effective amount of at least one iron chelating compound for a sufficient time to reduce the severity of a fungal condition, wherein the iron chelating compound comprises a non-siderophore or non-xenosiderophore relative to the fungal condition.

The invention additionally provides a method treating or preventing a fungal condition that includes administering to an individual having, or susceptible of having, a fungal condition a therapeutically effective amount of at least one iron chelating compound and at least one antifungal agent for sufficient time to reduce the severity of the fungal condition, wherein the iron chelating compound comprises a non-siderophore or non-xenosiderophore relative to the fungal condition.

The methods of the invention include iron chelation therapy alone or iron chelation therapy used together with antifungal therapy. In the former method, a formulation including at least one iron chelating compound is administered. In the latter method, a formulation including at least one iron chelating compound and at least one antifungal agent is administered. Such formulations are selected and prepared as described previously with respect to the compositions of the invention. Therefore, a composition of the invention can be prepared devoid of an at least one antifungal agent for use in iron chelation therapy alone or it can be prepared with at least one antifungal agent for use in combined iron chelation and antifungal therapy. A first composition of the invention also can be prepared devoid of at least one antifungal agent and a second composition of the invention can be prepared devoid of an iron chelating compound. The first and second preparations can then be used simultaneously, sequentially or alternatively for combination iron chelation and antifungal therapy. Therefore, the teachings and guidance described previously with respect to a composition of the invention can be similarly employed for selection and preparation of a formulation containing at least one iron chelating compound alone, a formulation containing at least one antifungal agent or for a formulation containing both.

For use in the methods of the invention, the iron chelating compound in a formulation containing at least one iron chelating compound, a first or second preparation containing at least one iron chelating compound and/or a composition of the invention will be selected so as to include a non-siderophore or non-xenosiderophore relative to the fungal condition or fungal agent causing the condition.

The term "siderophore," as it is used herein is intended to mean an iron chelator that facilitates iron gathering by an organism. For example, under conditions of iron starvation, many fungi synthesize siderophores that function in iron gathering through iron binding and uptake. Siderophores are generally low molecular weight compounds (e.g., less than about 2,000 MW) and can exhibit either or both cellular uptake and/or iron storage functions. Siderophores are synthesized by the utilizing organism. As compared to the term "iron chelator," which is generally used without reference to organism or species specificity, the term "siderophore" as it is used herein refers to an iron chelator in context with, or relative to, the siderophore-producing and utilizing organism or species. Accordingly, although iron chelating siderophores bind and decrease iron levels from the extracellular environment, because they facilitate iron uptake and use by a pathogen they have diminished therapeutic value when used for iron chelation therapy targeting a condition caused by the siderophore-producing organism. Siderophores synthesis and use can be found described in, for example, Howard, D.H., Clinical Microbiology Reviews 12:394-404 (1999).

The term "xenosiderophore," as it is used herein is intended to mean a siderophore not produced by the utilizing fungus or organism. The term "xenosiderophore" refers to an iron chelator in context with, or relative to, the xenosiderophore utilizing organism or species. Similar to siderophores, xenosiderophores exhibit therapeutic value when used for iron chelation therapy that targets a condition caused by a non-utilizing organism. Siderophore and xenosiderophore synthesis and use can be found described in, for example, Howard, D.H., FEMS Immunology and Medical Microbiology 40:95-100 (2004).

Therefore, an iron chelating compound corresponding to a non-siderophore or a non-xenosiderophore relative to a targeted fungal condition refers to a siderophore not produced or utilized by the fungal agent causing the fungal condition or to a xenosiderophore not utilized by the fungal agent causing the fungal condition.

The iron chelation formulations, compositions, including first and second constituent preparations, and methods of the invention are applicable for treating, reducing the severity, preventing and curing a fungal condition. A particularly useful application of the iron chelation formulations, compositions and methods of the invention include prophylactic administration prior to onset of the fungal condition.

The term "treating" or "treatment," as it is used herein is intended to mean an amelioration of a clinical symptom indicative of a fungal condition. Amelioration of a clinical symptom includes, for example, a decrease or reduction in at least one symptom of a fungal condition in a treated individual compared to pretreatment levels or compared to an individual with a fungal condition. The term "treating" also is intended to include the reduction in severity of a pathological condition, a chronic complication or an opportunistic fungal infection which is associated with a fungal condition. Such pathological conditions, chronic complications or opportunistic infections are exemplified below with reference to mucormycosis. Mucormycosis and other such pathological conditions, chronic complications and opportunistic infections also can be found described in, for example, Merck Manual, Sixteenth Edition, 1992, and Spellberg et al., Clin. Microbio. Rev. 18:556-69 (2005).

Symptoms of a fungal condition that can be ameliorated by a method of the invention include, for example, fever, chills, night sweats, anorexia, weight loss, malaise, depression and lung, skin or other lesions. Other symptoms or characteristic manifestations include, for example, dissemination from a primary focus, acute or subacute presentations, progressive pneumonia, fungemia, manifestations of extrapulmonary dissemination, chronic meningitis, progressive disseminated histoplasmosis as a generalized involvement of the reticuloendothelial system (liver, spleen, bone marrow) and blastomycosis as single or multiple skin lesions. Effective treatment of an individual with a fungal condition, for example, will result in a reduction one or more of such symptoms in the treated individual. Numerous other clinical symptoms of fungal conditions are well known in the art and also can be used as a measure of amelioration or reduction in the severity of a fungal condition using the methods of the invention described herein.

Diagnosis of a fungal condition can be confirmed by isolating causative fungi from, for example, sputum, urine, blood, bone marrow, or specimens from infected tissues. For example, fungal infections can be diagnosed histopathologically with a high degree of reliability based on distinctive morphologic characteristics of invading fungi and/or by immunohistochemistry and the like selective for identifying antigens. Assessment of the activity of the infection also can be based on cultures taken from many different sites, fever, leukocyte counts, clinical and laboratory parameters related to specific involved organs (eg, liver function tests), and immunoserologic tests. The clinical significance of positive sputum cultures also can be corroborated by confirmation of tissue invasion. Such corroboration can be particularly beneficial with commensal organisms such as *Candida albicans* or for those organisms that are prevalent in the environment such as *Aspergillus sp.*

The term "preventing" or "prevention," as it is used herein is intended to mean a forestalling of a clinical symptom indicative of a fungal condition. Such forestalling includes, for example, the maintenance of normal physiological indicators in an individual at risk of infection by a fungus or fungi prior to the development of overt symptoms of the condition or prior to diagnosis of the condition. Therefore, the term "preventing" includes the prophylactic treatment of individuals to guard them from the occurrence of a fungal condition. Preventing a fungal condition in an individual also is intended to include inhibiting or arresting the development of the fungal condition. Inhibiting or arresting the development of the condition includes, for example, inhibiting or arresting the occurrence of abnormal physiological indicators or clinical symptoms such as those described above and/or well known in the art. Therefore, effective prevention of a fungal condition would include maintenance of normal body temperature, weight, psychological state as well as lack of lesions or other pathological manifestations as described above in an individual predisposed to a fungal condition. Individuals predisposed to a fungal condition include, for example, an individual with AIDS, azotemia, diabetes mellitus, bronchiectasis, emphysema, TB, lymphoma, leukemia, or bums, or an individual with a history of susceptibility to a fungal condition. Inhibiting or arresting the development of the condition also includes, for example, inhibiting or arresting the progression of one or more pathological conditions, chronic complications or susceptibility to an opportunistic infection associated with a fungal condition.

The methods of the invention are useful for the treatment and/or prevention of a wide variety of fungal conditions. The term "fungal condition," as it is used herein is intended to mean an aberrant condition causes by a fungus infection. Fungal infection, or mycoses, of humans and animals include, for example, superficial fungal infections that affect the outer layers of skin; fungal infections of the mucous membranes including the mouth (thrush), vaginal and anal regions, such as those caused by *Candida albicans,* and fungal infections that affect the deeper layers of skin and internal organs are capable of causing serious, often fatal illness. Fungal infections are well known in the art and include, for example, zygomycosis, aspergillosis, cryptococcosis, candidiasis, histoplasmosis, coccidiomycosis, paracoccidiomycosis, fusariosis (hyalohyphomycoses), blastomycosis, penicilliosis or sporotrichosis. These and other fungal infections can be found described in, for example, Merck Manual, Sixteenth Edition, 1992, and in Spellberg et al., Clin. Microbio. Rev. 18:556-69 (2005). The exemplary fungal conditions described above are described further below. As used herein, the term "zygomycosis" is intended to mean a fungal condition caused by fungi of the class *Zygomycetes,* comprised of the orders Mucorales and Entomophthorales. The Entomophthorales are causes of subcutaneous and mucocutaneous infections known as entomophthoromycosis, which largely afflict immunocompetent hosts in developing countries.

As used herein, the term "mucormycosis" is intended to mean a fungal condition caused by fungi of the order Mucorales. Mucormycosis is a life-threatening fungal infection almost uniformly affecting immunocompromised hosts in either developing or industrialized countries. Fungi belonging to the order Mucorales are distributed into six families, all of which can cause cutaneous and deep infections. Species belonging to the family Mucoraceae are isolated more frequently from patients with mucormycosis than any other family. Among the Mucoraceae, *Rhizopus oryzae* (*Rhizopus arrhizus*) is a common cause of infection. Other exemplary species of the Mucoraceae family that cause a similar spectrum of infections include, for example, *Rhizopus microsporus* var. *rhizopodiformis, Absidia corymbifera, Apophysomyces elegans, Mucor* species, *Rhizomucor pusillus* and *Cunninghamella* spp (*Cunninghamellaceae* family). Mucormycosis is well known in the art and includes, for example, rinocerebral mucormycosis, pulmonary mucormycosis, gastrointestinal mucormycosis, disseminated mucormycosis, bone mucormycosis, mediastinum mucormycosis, trachea mucormycosis, kidney mucormycosis, peritoneum mucormycosis, superior vena cava mucormycosis or external otitis mucormycosis.

As used herein, the term "candidiasis" is intended to mean a fungal condition caused by fungi of the genus *Candida.* Candidiasis can occur in the skin and mucous membranes of the mouth, respiratory tract and/or vagina as well as invade the bloodstream, especially in immunocompromised individuals. Candidiasis also is known in the art as candidosis or moniliasis. Exemplary species of the genus *Candida* include, for example, *Candida albicans, Candida krusei, Candida tropicalis, Candida glabrata* and *Candida parapsilosis.*

As used herein, the term "aspergillosis" is intended to mean the group of diseases caused by the genus *Aspergillus.* The symptoms include, for example, fever, cough, chest pain and/or breathlessness. Patients with a weakened immune systems or who suffer from a lung condition are particularly susceptible to aspergillosis. Exemplary forms of this fungal condition includeallergic aspergillosis, which affects asthma, cystic fibrosis and sinusitis patients); acute invasive aspergillosis, which shows increased incidence in patients with weakened immunity such as in cancer patients, patients undergoing chemotherapy and AIDS patients; disseminated invasive aspergillosis, which is widespread throughout the body, and opportunistic *Aspergillus* infection, which is characterized by inflammation and lesions of the ear and other organs. *Aspergillus* is a genus of around 200 fungi. *Aspergillus* species causing invasive disease include, for example, *Aspergillus fumigatus* and *Aspergillus flavus. Aspergillus* species causing allergic disease include, for example, *Aspergillus fumigatus* and *Aspergillus clavatus.* Other exemplary *Aspergillus* infectious species include, for example, *Aspergillus terreus* and *Aspergillus nidulans.*

As used herein, the term "cryptococcosis" is intended to mean a fungal condition caused by the genus *Cryptococcus.* Cryptococcosis, also known as Busse-Buschke disease, generally manifests as a systemic infection that can affect any organ of the body including, for example, the lungs, skin, or other body organs, but most often occurs in the central nervous system such as the brain and meninges. Cryptococcosis is an opportunistic infection for AIDS, although patients with Hodgkin's or other lymphomas or sarcoidosis or those receiving long-term corticosteroid therapy are also at increased risk. Symptoms include, for example, chest pain, dry cough, swelling of abdomen, headache, blurred vision and confusion. Exemplary forms of this fungal condition include cutaneous cryptococcosis such as those occurring in wounds, pulmonary cryptococcosis and Cryptococcal meningitis. Cryptococcal meningitis can result from dissemination of *Cryptococcus neoformans* from either an observed or unappreciated pulmonary infection generally in immunocompromised patients *C. gattii* generally causes infections in immunocompetent people. Detection of cryptococcal antigen (capsular material) by culture of CSF, sputum and urine provides one useful method of diagnosis. Blood cultures also can be positive in heavy infections.

As used herein, the term "histoplasmosis" is intended to mean a fungal condition caused by the genus *Histoplasma,* including the infectious disease caused by the inhalation of spores of *Histoplasma capsulatum.* Histoplasmosis also is known in the art as Darling's disease. The condition can be asymptomatic, but also can progress to acute pneumonia or an influenza like illness, primarily affects the lungs. Histoplasmosis also can spread to other organs and systems in the body. As with other disseminated forms of fungal conditions, this disseminated histoplasmosis can be fatal. Symptoms can occur within 3 to 17 days after exposure. However, in undisseminated forms, it can be common for infected individuals to exhibit no apparent ill effects. The acute respiratory disease can be characterized by respiratory symptoms, a general ill feeling, fever, chest pains, and a dry or nonproductive cough. Distinct patterns also can be seen on a chest x-ray. Chronic lung disease resembles tuberculosis and can worsen over months or years.

As used herein, the term "coccidiomycosis" is intended to mean a fungal condition caused by the genus *Coccidioides.* Included in the meaning ofthe term is the infectious respiratory disease caused by *Coccidioides immitis* or *C. posadasii,* particularly through inhalation of spores, and which is characterized by fever and various respiratory symptoms. Coccidiomycosis also is known in the art as coccidioidomycosis and valley fever. Systemic coccidiomycosis can spread from the respiratory tract to, for example, the skin, bones, and central nervous system. Manifestations of the condition range from complete absence of symptoms to systemic infection and death. For example, symptomatic infection (about 40% of cases) can present as an influenza-like illness with fever, cough, headaches, rash, and myalgia (muscle pain). Some patients can fail to recover and develop chronic pulmonary infection or widespread disseminated infection (affecting meninges, soft tissues, joints, and bone). Severe pulmonary disease can develop in, for example, HIV-infected and other immunocompromised persons.

As used herein, the term "paracoccidiomycosis" is intended to mean a fungal condition caused by the genus *Paracoccidioides* including, for example, a chronic mycosis caused by *Paracoccidioides brasiliensis.* Paracoccidiomycosis is characterized by primary lesions of the lungs with dissemination to many internal organs, by conspicuous ulcerative granulomas of the mucous membranes of the cheeks and nose with extensions to the skin, and by generalized lymphangitis. Paracoccidiomycosis also is known in art as paracoccidioidomycosis, Almeida's disease, Lutz-Splendore-Almeida disease, paracoccidioidal granuloma and South American blastomycosis.

As used herein, the term "fusariosis" or ""hyalohyphomycoses" is intended to mean a fungal condition caused by the *genus fusarium.* Fusarium species causing the condition include, for example, *F. solani, F. oxysporum* and *F. moniliforme.* Infections include keratitis, onychornycosis and occasionally peritonitis and cellulitis. Risk factors for disseminated fusariosis include severe immunosuppression (neutropenia, lymphopenia, graft-versus-host disease, corticosteroids), colonisation and tissue damage. Among immunocompetent patients, tissue breakdown (as caused by trauma, severe burns or foreign body) is the risk factor for fusariosis. Clinical presentation includes refractory fever, skin lesions and sinopulmonary infections. Skin lesions can lead to diagnosis in many patients and precede fungemia by approximately 5 days. Disseminated fusariosis can be diagnosed by, for example, blood cultures and other well known methods described above and below.

As used herein, the term "blastomycosis" is intended to mean a fungal condition caused by the genus *blastomycete,* generally originating as a respiratory infection, and usually spreading to the lungs, bones, and skin. Blastomycosis is characterized by multiple inflammatory lesions ofthe skin, mucous membranes, or internal organs. *Blastomyces dermatitidis* is one species prevalent causative species. Symptoms of blastomycosis include, for example, a flulike illness with fever, chills, myalgia, headache, and a nonproductive cough; an acute illness resembling bacterial pneumonia, with symptoms of high fever, chills, a productive cough, and pleuritic chest pain; a chronic illness that mimics tuberculosis or lung cancer, with symptoms of low-grade fever, a productive cough, night sweats, and weight loss; a fast, progressive, and severe disease that manifests as ARDS, with fever, shortness of breath, tachypnea, hypoxemia, and diffuse pulmonary infiltrates; skin lesions; bone lytic lesions; prostatitis, and/or laryngeal involvement causing hoarseness.

As used herein, the term "penicilliosis" is intended to mean a fungal condition caused by the *genus penicillium.* An exemplary species *is penicillium marneffei,* which is a prevalent cause of opportunistic fungal infections in immunocompromised individuals. Diagnosis is can be made by identification of the fungi from clinical specimens. Biopsies of skin lesions, lymph nodes, and bone marrow can demonstrate the presence of organisms on histopathology. Symptoms include, for example, fever, skin lesions, anemia, generalized lymphadenopathy, and hepatomegaly.

As used herein, the term "sporotrichosis" is intended to mean a fungal condition caused by the genus *Sporothrix,* including the species *S. schenckii.* The condition manifests as a chronic infectious characterized by nodules or ulcers in the lymph nodes and skin.

Sporotrichosis infection can spread through the blood to other areas including, for example, infection of the joints, lungs, eye, and the genitourinary and central nervous system. Generally, disseminated sporotrichosis occurs in immunocompromised individuals such as patients with AIDS, cancer, patients undergoing chemotherapy, and transplant recipients on immunosuppressive therapy.

As used herein, the terms "effective amount" or "therapeutically effective amount" are intended to mean an amount of an iron chelating compound, an antifungal agent or both an iron chelating compound and an antifungal agent of the invention required to effect a decrease in the extent, amount or rate of spread of a fungal condition when administered to an individual. Therefore, an effective amount when used in reference to an iron chelating compound is intended to mean an amount of the iron chelating compound sufficient to ameliorate at least one symptom associated with a targeted fungal condition.

The dosage of an iron chelating compound, an antifungal agent or both an iron chelating compound and an antifungal agent of the invention required to be therapeutically effective will depend, for example, on the fungal condition to be treated, the affinity of the iron chelating compound for iron, the level of abundance and concentration of iron as well as the weight and condition of the individual, and previous or concurrent therapies. The appropriate amount considered to be an effective dose for a particular application of the method can be determined by those skilled in the art, using the guidance provided herein. For example, the amount can be extrapolated from *in vitro* or *in vivo* assays as described below. One skilled in the art will recognize that the condition of the patient needs to be monitored throughout the course of therapy and that the amount of the composition that is administered can be adjusted according to the response of the therapy.

Exemplary effective amounts of iron chelating compounds include, for example, a concentration range from about 0.3-3.0 µg/ml, but also can include concentrations as low as 0.01 µg/ml or lower and as high as 10 µg/ml or higher. The amount of an antifungal agent to be administered are well known in the art and are included herein within the meaning ofthe term "effective amount."

In pre-clinical investigations of deferasirox, for example, virtually no toxicity was seen at doses up to 400 mg/kg/d in iron-overloaded marmosets (Nick et al. Adv. Exp. Med. Biol. 509:185-203 (2002)). However, severe toxicity was seen after chronic administration to non-iron-overloaded marmosets at doses of 80 mg/kg, confirming that deferasirox is less well tolerated in non-iron-overloaded hosts (*Id.*). Nevertheless, even in non-iron overloaded marmosets, no toxicities were seen after 39 weeks of treatment with up to 40 mg/kg/d, and during subacute treatment (4 weeks), doses up to 65 mg/kg/d were well tolerated with no adverse effects. In clinical investigations of iron overloaded patients (Nisbet-Brown et al. Lancet 361:1597-1602 (2003); Galanello et al. J. Clin. Pharmaco/. 43:565-572 (2003); Cappellini et al. Blood 107:3455-3462 (2006)), deferasirox was very well tolerated at doses up to 40 mg/kg/d for up to 12 days, and in chronic dosing, over months to years, at up to 30 mg/kg/d.

Generally, an iron chelating compound will be included in an iron chelation therapy formulation, a first or second constituent formulation and/or a composition of the invention at a concentration from between about 0.01-10 µg/ml, about 0.1-8 µg/ml,, about 0.2-6 µg/ml, particularly between about 0.3-3.0 µg/ml, more particularly between about 0.6-2.0 µg/ml,, even more particularly between about 0.8-1.0 µg/ml or about 0.9 µg/ml. Iron chelating compound concentrations and/or amounts less than, greater than or in between these ranges also can be used in an iron chelation therapy formulation, a first or second constituent formulation and/or a composition of the invention. For example, one or more iron chelating compounds can be included in a formulation or composition which constitute less than about 0.1 µg/ml. Similarly, a formulation or composition can contain a concentration of one or more iron chelating compounds greater than about 10 µg/ml, particularly when formulated for storage. Accordingly, a formulation or composition useful in the methods of the invention can be produced that contains essentially any desired concentration or amount of one or more iron chelating compounds including, for example, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 6, 7, 8, 9, 10, 11, 12 µg/ml or more.

Exemplary effective amounts of antifungal agents include, for example, those exemplified below with respect to polyene antifungal agents. For example, amphotericin B deoxycholate is in general administered at, for example, a dose of less than about 10 mg/kg of body weight and can include rates of about 0.25-1.0 mg/kg/day for intravenous administration. Liposomal amphotericin B (L-AMB) is in general administered at, for example, doses of 1-5 mg/kg. Liposomal amphotericin B can also be administered as high as 15 mg/kg/day especially in cerebral disease. Amphotericin B lipid complex (ABLC) is in general administered at a dose of 5 mg/kg, but can range from about 0.5-15 mg/kg. Amphotec is in general administered at single doses of about 50-100 mg. Similarly, for combination therapy using an iron chelating compound and an antifungal agent, the above amounts can be administered together or either or both the compound and/or the agent amount can be modulated up or down. Effective amounts for azole antifungal agents and echinocandin antifungal agents similarly are well known to those skilled in the art, and generally include dosages within the ranges above.

Given the teachings and guidance provided herein, those skilled in the art will understand that the effective amounts exemplified above can be modulated with respect to, for example, the type and amount of the other active ingredient. For example, if dosages of the at least one iron chelating compound are in the high range exemplified above, if desired, one can decrease the amount of one or more of the at least antifungal agent while still obtaining an effective amount. Similarity, if dosages of the at least one antifungal agent are in the high range or higher than those exemplified above, if desired, one can decrease the amount of one or more of the at least one or more of the at least iron chelating compounds. Dosages of two or more iron chelating compounds and/or dosages of two or more of the at least one antifungal agents also can be modulated with respect to each other. All other modulations of such combined effective amounts can be utilized in the method of the invention.

It is understood that modifications which do not substantially affect the activity of the various embodiments of this invention are also included within the definition of the invention provided herein. Accordingly, the following examples are intended to illustrate but not limit the present invention.

### EXAMPLE I

### Iron Chelation Therapy for the Reduction in Severity of Fungal Diseases

This Example describes preliminary studies showing that deferiprone and Exjade^{Tm} can reduce the pathogenesis of mucormycosis.

Animal models have demonstrated that administration of deferoxamine or free iron worsens survival of animals infected with *Rhizopus* but to a lesser extent *Candida albicans,* underscoring the unique requirement of iron for *Rhizopus* pathogenicity (Abe et al., Mycopathologia 110:87-91 (1990); Boelaert et al., (1993), *supra;* Boelaert et al., (1994), *supra;* Van Cutsem and Boelaert, Kidney International 36:1061-68 (1989)). Additionally, *in vitro* studies of radiolabeled iron uptake from deferoxamine in serum showed that *Rhizopus* accumulated 8-fold and 40-fold greater amounts of iron than did *Aspergillus fumigatus* and *C. albicans,* respectively (Boelaert et al., (1993), *supra*). This increased iron uptake by *Rhizopus* was linearly correlated with its growth in serum (Boelaert et al., (1993), *supra*). The increased growth in serum may explain the special affinity of Zygomycetes to directly penetrate and grow through blood vessels, which results in the propensity for thrombosis and tissue necrosis during the disease (Edwards, (1989), *supra;* Ibrahim et al., (2003), *supra;* Ventura et al., *supra*).

As described previously, patients with diabetic ketoacidosis are also at high risk of developing rhinocerebral mucormycosis. These patients also have elevated levels of available serum iron, likely due to release of iron from binding proteins in the presence of acidosis (Artis et al., *supra*). Sera collected from patients with diabetic ketoacidosis supported growth of *R. oryzae* in the presence of acidic pH (7.3-6.88) but not in the presence of alkaline pH (7.78-8.38). Sera that did not support *R. oryzae* growth at acidic pH were found to have less available iron than sera that supported fungal growth. Furthermore, adding exogenous iron to sera allowed *R. oryzae* to grow profusely at acidic conditions but not at pH > 7.4. Finally, simulated acidotic conditions decreased the iron-binding capacity of sera collected from normal volunteers, suggesting that acidosis temporarily disrupts the capacity of transferrin to bind iron. Therefore, the increased susceptibility to mucormycosis of patients with diabetic ketoacidosis is likely due at least in part to an elevation in available serum iron during diabetic ketoacidosis.

The role of iron metabolism in the pathogenesis of mucormycosis allows the possibility of utilizing effective iron chelators for antifungal therapy, including as an adjunctive antifungal therapy. In contrast to deferoxamine, other iron chelators have not allowed the organism to take up iron, and do not support its growth *in vitro* in the presence of iron (see, for example, Boelaert et al., (1994), *supra*). Furthermore, while deferoxamine significantly worsened disseminated *R. oryzae* infection in guinea pigs, one of the other chelators had no impact on *in vivo* infection and the other chelator more than doubled the mean survival time of infected guinea pigs (Table 1).

**Table 1. Effects of iron chelators on survival of guinea pigs infected with R. oryzae.**

| **Treatment** | **Mean (± SD) Survival Time (days)** |
|---|---|
| Saline | 7.8 ± 1.1 |
| Deferoxamine | 4.8 ± 1.1 |
| Deferiprone | 16.2 ± 4.0 |
| CP94 | 8.7 ± 1.0 |
| *p < 0.05 vs. saline control. | |

*In vitro* and *in vivo* data described herein indicate that deferiprone can be used to effectively treat mucormycosis infections. Table 1 shows the results of a preliminary study where deferiprone significantly increases the mean survival time of infected animals. These preliminary studies further showed that that deferiprone was highly active *in vitro* against *R. oryzae,* showing static effect at 24 h but cidal effect at 48 h of incubation [minimum inhibitory concentration (MIC) and minimum fungicidal concentration (MFC) of 6.25 µg/ml for both after 48 h]. In infected DKA mice, Liposomal amphotericin B (LAmB) which is a general therapy for mucormycosis or deferiprone (Def) improved 28-day survival compared to placebo (52% survival for LAmB vs. 30% for Def vs. 0% for placebo, p < 0.003 by Log Rank test for LAmB or Def vs. placebo; p = 0.15 for LAmB vs. Def). The efficacy of Def was abrogated by administration to the mice of FeCl₃, confirming the mechanism of Defmediated protection is iron chelation (Figure 1). This latter agent, deferiprone, is approved for clinical use as an iron chelator in Europe and India, and is available on a compassionate use basis for iron overload in the United States.

Exjade^{Tm} (Novartis), which is another iron chelator, has been approved for clinical use in iron overload patients in the United States. This drug has been tested against *R. oryzae in vitro* and in animal models using a diabetic ketoacidotic mouse model. Mice infected with a lethal dose of *R. oryzae* and treated with Exjade^{Tm} at 1, 3, or 10 mg/kg bid survived longer than placebo treated mice [28 day survival days was 0% survival for Placebo vs. 29% survival for 1 mg/kg Exjade® (p=0.03), vs. 40% survival for mice treated with 3 mg/kg Exjade® (p=0.049) vs. 57% survival of mice treated with 10 mg/kg Exjade® (p=0.01) by Log Rank test] (Figure 2). These studies demonstrate the efficacy of using Exjade^{Tm} in treating mucormycosis. These studies additionally indicate that the above iron chelators can be applicable to the treatment of other fungal infections, including mucormycosis in neutropenic individuals as well as other infections such as disseminated *candidiasis* and *aspergillosis.*

### EXAMPLE II

### Iron Chelation Therapy for the Treatment of Mucormycosis

This Example shows a comparison of the efficacy of deferiprone to liposomal amphotericin B (LAmB) in treating mucormycosis in diabetic ketoacidotic (DKA) mice or prophylaxing against mucormycosis in neutropenic mice.

*R. oryzae* is a common cause of mucormycosis (Ibrahim et al., (2003), *supra*). Patients with elevated available serum iron, such as diabetics in ketoacidosis, are at high risk of developing mucormycosis infections (Artis et al., *supra*). Additionally, deferoxamine iron-chelation therapy predisposes patients to these infections. Deferoxamine acts as a xeno-siderophore to supply previously unavailable iron to the fungus (Boelaert et al., (1993) *supra*). Therefore, the use of an iron-chelator that cannot be utilized by the fungus to scavenge iron from the host was assessed for efficacy against mucormycosis. Def is an iron chelator which cannot be utilized by *Rhizopus* to scavenge iron (Boelaert et al., (1994) *supra*).

Initially, *the in vitro* activity of deferiprone against *R. oryzae* was assessed in this study. Briefly, *R. oryzae* 99-880 was isolated from a brain abscess of a diabetic patient. The organism was grown on potato dextrose agar (PDA) for 3 days at 37°C. In some experiments, *R. oryzae* was starved for iron by growth on PDA in the presence of 1 mM of ascorbic acid and ferrozine. The sporangiospores were collected in endotoxin free PBS containing 0.01% Tween 80, washed with PBS then counted with hemacytometer to prepare the final inocula. For susceptibility testing, the minimum inhibitory concentration (MIC) and minimum fungicidal concentration (MFC) were determined for Def and deferoxamine by the method of Espinel-Ingroff (J Clin Microbiol 39:954 (2001)).

The results are shown in Table 2 below. Deferiprone was static against *R. oryzae* at 24 h (MIC and MFC = 3.12 and 100 µg/ml, respectively), but demonstrated cidality at 48 h of incubation (MIC and MFC = 6.25 µg/ml). In contrast, deferoxamine, which is known to supply iron to *R. oryzae,* did not inhibit the growth of *R. oryzae* (MIC and MFC of > 100 µg/ml after 24 or 48 hr), and in fact, by visual inspection, growth in wells containing deferoxamine was greater than in the growth control wells (containing no iron chelators).

**Table 2: MIC90 and MFC of DEF and deferoxamine against R. oryzae. Values are expressed in mg/ml.**

| Time (h) | Deferiprone | | Deferoxamine | |
|---|---|---|---|---|
| | MIC | MFC | MIC | MFC |
| 24 | 3.12 | 100 | >100 | >100 |
| 48 | 6.25 | 6.25 | >100 | >100 |

Having confirmed its *in vitro* activity against *R. oryzae,* we used our DKA mouse model to evaluate the role of deferiprone in treating disseminated *R. oryzae* infection *in vivo.* Briefly, for *in vivo* infection, BALB/c male mice (≥20g) were rendered diabetic with a single i.p. injection of 210 mg/kg streptozotocin in 0.2 ml citrate buffer 10 days prior to fungal challenge. Glycosuria and ketonuria were confirmed in all mice 7 days after streptozotocin treatment. Mice were infected through the tail vein with the appropriate inocula of *R. oryzae.* To confirm the inocula, dilutions were streaked on PDA plates and colonies were counted following a 24 h incubation period at room temperature. The primary efficacy endpoint was time to death. As a secondary endpoint, brain fungal burden (the primary target organ) was determined by homogenization by rolling a pipette on organs placed in Whirl-Pak bags (Nasco, Fort Atkinson, WI) containing 2 ml saline. The homogenate was serially diluted in 0.85% saline and then quantitatively cultured on PDA. Values were expressed as log10 cfu g⁻¹ tissue. All procedures involving mice were approved by the institutional animal use and care committee, following the National Institutes of Health guidelines for animal housing and care.

Regarding drugs and therapy regimens, LAmB diluted in 5% dextrose water was obtained from Gilead Sciences and was administered at 15 mg/kg qd (once a day) intravenously through the tail vein. Deferiprone (Apotex Research Inc.) was dissolved in iron-free water and administered intraperitoneally at 50, 100, or 200 mg/kg qd or qod (every other day). Treatment was begun 24 h post infection and continued for a total of 4 doses. Control groups were treated with the diluent, 5% dextrose water.

In some experiments, a saturating dose of free iron was administered with deferiprone, in an attempt to reverse the efficacy of iron-chelation. Deferiprone is known to form molecular complexes with ferric iron (³⁺Fe) in a 1:3 ratio of iron to deferiprone. Based on the known molecular weights of ferric chloride (FeCl₃, molecular weight 162.22 g/mol) and deferiprone (molecular weight 139 g/mol), a 60 mg/kg dose of FeCl₃ was calculated to result in a significant excess of ³⁺Fe vs. a 100 mg/kg dose of deferiprone given to an 18 g mouse: ³⁺Fe moles = (0.060 g/kg * 0.018 kg [mouse weight]/ 162.22 g/mol) = 6.5 x 10⁻⁶ mol vs. deferiprone moles = (0.1 g/kg * 0.018 kg [mouse weight]/139 g/mol) = 1.3 x 10⁻⁵ mol Ratio = 1:2 moles of ³⁺Fe to deferiprone, which is more than that required to achieve a saturating 1:3 ratio.

Statistical analysis was performed using the nonparametric log rank test was used to determine differences in survival times of the mice. Differences in tissue fungal burdens in the infected organs were compared by the nonparametric Steel test for multiple comparisons. P values of <0.05 were considered significant.

Initially, a dose response was performed, using doses based on unpublished observations from the manufacturer (50, 100, or 200 mg/kg of deferiprone administered qd or qod). Mice were infected with 4.3 x 10³ spores of *R. oryzae* and deferiprone treatment was initiated 24 h post infection and continued for a total of four doses. The results are shown in Figures 3 and 1, which indicate that deferiprone protected DKA mice from R. oryzae infection. A deferiprone dose of 100 mg/kg qod improved survival ofDKA mice compared to placebo (*P*=0.027, Figure 3). Higher doses, including 100 mg/kg qd or 200 mg/kg qd or qod, did not improve survival, nor did the 50 mg/kg qd or qod doses.

As described previously in Example I, efficacy of deferiprone also was compared to a general treatment of mucormycosis, high dose LAmB. A dose of 15 mg/kg of LAmB administered qd was chosen because we demonstrated that this dose is more protective in our DKA mouse model than 1 mg/kg amphotericin B deoxycholate. Furthermore, to confirm that the mechanism of protection of deferiprone was chelation of iron, the efficacy of deferiprone plus a saturating dose (60 mg/kg) of free iron in the form of FeCl₃ was also evaluated. FeCl₃ was administered intravenously every time deferiprone was given to the animals.

LAmB or deferiprone at 100 mg/kg qod improved 28-day survival compared to placebo (Figure 1, *P*< 0.003 by Log Rank test for LAmB or deferiprone vs. placebo). There was no statistical difference in survival of mice treated with LAmB vs. mice treated with deferiprone (*P* = 0.15). The efficacy of deferiprone was completely abrogated by administration of ferric chloride (Figure 1).

In additional studies, treatment of experimental mucormycosis with a combination of Def and LAmB also was assessed. Briefly, mice (n=11, for infected untreated and Def and n=6 LAmB and Def + LAmB) were infected with *R. oryzae* and then treated 24 h later with Def, LAmB, or a combination of both drugs for a total of 4 doses. The results are shown in Figure 4 and indicate enhanced efficacy using a combination of both treatments. (*, p<0.005 compared to infected untreated mice).

Similarly, the survival of neutropenic mice infected with *R. oryzae* and prophylaxed with Def, LAmB, or both also was assessed. Mice (n=9) were treated on day -1 with Def qod, LAmB qd, or both for a total of four doses. These results are shown in Figure 5 and also indicate enhanced efficacy using a combination of anti-fungal treatment combined with iron chelation. (*p < 0.05 for LAmB or LAmB + Def vs. infected untreated). For example, prophylactic Def resulted in 22% survival vs. 0% survival in placebo-treated mice (p > 0.05); both LAmB and LAmB + Def resulted in 100% survival (p < 0.05 vs. placebo for both).

As an additional marker of efficacy, the impact of deferiprone therapy on brain fungal burden also was evaluated, as the brain is the primary target organ in this animal model. Mice were infected with 3.8 x 10³ spores then treated with two doses of LAmB (qd) or deferiprone (qod). In the above studies, control mice began to die before the second dose of deferiprone had been administered. To enable testing of at least two doses of every-other-day deferiprone prior to determining tissue fungal burden, in this study deferiprone was administered at 30 minutes and 48 h post infection, whereas LAmB was given 24 and 48 h post infection. Brains were harvested approximately 54 h post-infection. The results are shown in Figure 6 and indicate that both drugs reduced brain fungal burden compared to placebo (*P* ≤ 0.036). The above results indicate that Def iron chelation therapy is effective in treating experimental murine mucormycosis and also can have prophylactic efficacy, including in the neutropenic setting. In particular, Def inhibited the growth of *R. oryzae in vitro*, whereas deferoxamine dose not. Def has considerable efficacy in treating mucormycosis in the DKA model and this efficacy is comparable to high dose therapy of LAmB. The efficacy of Def is likely due to its ability to chelate iron since administration of free iron reversed its protection. Def prophylaxis did not appear to antagonize the protective effect of LAmB in the neutropenic mouse model of mucormycosis.

### EXAMPLE III

### Deferasirox Administration for the Therapeutic Treatment of Mucormycosis

This Example shows the use ofDeferasirox, an iron-chelating agent, as a salvage therapy for rhinocerebral mucormycosis.

Deferasirox (Exjade®) is a novel, first-in-class, orally available iron chelator, recently approved by the United States (US) Food and Drug Administration (FDA) for the treatment of iron overload in transfusion-dependent anemias. This study describes the use of deferasirox as salvage therapy for a patient with rhinocerebral mucormycosis failing maximum conventional antifungal therapy.

A 40 year old man with diabetic ketoacidosis presented to the emergency department of an outside hospital with polyuria, polydipsia, left retrobulbar pain, and a left cranial nerve (CN) VI palsy. Computed tomography (CT) scan of the head showed only left sphenoid and ethmoid sinusitis. The patient progressed to complete left ophthalmoplegia in the first 24 hours. Rhinocerebral mucormycosis was suspected, and he was started on amphotericin B lipid complex (ABLC) at 5 mg/kg/day, along with empiric antibacterial agents, for treatment of sinusitis. On hospital day 2 he underwent endoscopic sphenooethmoid exploration. No necrotic tissue or eschar was found, and multiple biopsies showed nonspecific inflammation. Fungal culture was negative and special stains revealed no organisms. Bacterial culture grew only rare *Enterobacter aerogenes.*

Over the next twelve days, the patient developed blindness and worsening proptosis in the left eye. He was continued on ABLC and no further surgery was performed. Serial magnetic resonance imaging (MRI) of the orbits and brain revealed progression of left-sided pansinusitis, and thickening and abnormal enhancement of the left extraocular muscles. The patient was transferred to our institution for further management on hospital day 14. Immediately upon arrival, he underwent left orbital exenteration with enucleation and extensive sinus debridement and resection. Intraoperative findings included an ischemic globe with extensive surrounding necrosis of the extraocular muscles. Pathologic specimens showed evidence of Mucorales (Figure 7); fungal cultures were negative. The patient was started on liposomal amphotericin B (LamB) at 15 mg/kg/day and caspofungin at 70 mg/kg/d (based on published mouse data demonstrating polyene-echinocandin synergy). Head CT scan performed two days after the orbital exenteration showed left cavernous sinus thrombosis; this finding was confirmed on follow up MRI.

Despite the absence of new neurological findings, an MRI on hospital day 36 showed progression of left cavernous sinus thrombosis with slow flow in the left internal carotid and new enhancement of CN V consistent with progressive mucormycosis. Antifungal therapy was continued. On hospital day 71, a brain MRI showed new enhancement in the suprasellar cistern and, for the first time, the development of a brainstem lesion in the pons with surrounding edema. Given the location of the lesion at the superficial aspect of the pons, a dose of intracisternal amphotericin was administered, but the patient refused further doses. The patient remained clinically and neurologically unchanged. Due to stabilization of subsequent imaging, he was discharged on hospital day 86 to continue outpatient LAmB monotherapy three times per week.

His disease remained clinically and radiographically stable for several months. Because of worsening renal insufficiency (Cr 3.5-4.0, from a baseline of 0.8) ten weeks after discharge, the dose of LAmB was reduced to 10 mg/kg twice weekly, and then titrated down to once weekly four months after discharge. One month later, repeat MRI showed new enhancement with surrounding edema in the left middle cerebellar peduncle and the left cerebellopontine angle consistent with progressive disease.

Based on data from the experimental iron chelator, deferiprone, as well as unpublished observations of deferasirox in mice, deferasirox was administered at a dose of 1000 mg po (by mouth) daily for seven days. LAmB was continued. On the last day of deferasirox treatment, the patient developed an erythematous, slightly pruritic, confluent maculopapular eruption over the trunk and extremities consistent with a probable drug rash. The pruritis was managed with diphenhydramine. No other adverse events were noted. Repeat MRI of the brain one week after deferasirox treatment demonstrated resolution of the edema in the left cerebellar peduncle. Follow up imaging five weeks later showed no new lesions. Because of worsening renal insufficiency (Cr ∼ 5.5), and in the setting of stability of the radiographic findings on repeat imaging, LAmB was discontinued. Ten months after his initial diagnosis, the patient remained asymptomatic and neurologically intact.

The patient described here was clearly not cured of his invasive mucormycosis despite extensive surgery and months of antifungal therapy, because he developed new radiographic findings consistent with progressive mucormycosis after 7 months of treatment with maximum tolerated doses of LamB. However, after 7 days of treatment with deferasirox, a substantial radiographic improvement was noted. Two months after deferasirox was administered, all antifungal therapy was discontinued. The patient remained clinically asymptomatic and radiographically stable afterwards, with no sign of disease progression. The patient tolerated deferasirox well aside from a minor drug reaction that may be attributable to the chelator.

Here we describe a case of advanced mucormycosis, with brainstem and cavernous sinus involvement after a marked delay in appropriate surgery, with an apparently successful outcome after the use of deferasirox for salvage therapy. The precise effect of the deferasirox treatment is confounded somewhat by concurrent polyene therapy. However the patient had clearly failed very aggressive polyene therapy before addition of the deferasirox. Given the encouraging results in this case, and the limited options and poor prognosis of mucormycosis despite current antifungal therapies, additional study of iron chelation in treatment of mucormycosis is warranted.

### EXAMPLE IV

### The Effects of Deferasirox on Mucorales in vitro

This Example shows the effects of deferasirox on expression of the high affinity iron permease gene (rFTR1) in *Rhizopus oryzae.* This example also shows the deferasirox susceptibilities of multiple clinical isolates of Mucorales.

The following describes materials and methods used in the procedures described in this or the subsequent examples.

### R. oryzae and culture conditions

*R. oryzae* 99-880 is a clinical isolate from a brain abscess of a diabetic patient. *R. oryzae* 99-892 is a clinical, pulmonary isolate. *R. oryzae* M16 is a *pyrF* null mutant (pyrF encodes orotidine 5'-phosphate decarboxylase, an enzyme required for uracil synthesis) generated from *R. oryzae* 99-880 by chemical mutagenesis. This mutant exhibited a stable phenotype for uracil auxotrophy even when plating more than 1x10⁹ spores. Organisms were grown on potato dextrose agar (PDA) [formulation/liter; potato starch 4g, dextrose 20g, agar 15g] for 4 days at 37°C. For M16, PDA was supplemented with 100 µg/ml uracil. In some experiments, *R. oryzae* was starved for iron by growth on yeast nitrogen base (YNB) (Difco/Becton Dickinson, Sparks, MD) supplemented with complete supplemental media without uracil (CSM-URA), (YNB+CSM-URA) [formulation/100ml, 1.7g YNB without amino acids, 20g glucose, 0.77g CSM-URA] in the presence of 1 mM of ascorbic acid and ferrozine. The sporangiospores were collected in endotoxin free PBS containing 0.01% Tween 80, washed with PBS, and counted with a hemacytometer to prepare the final inocula.

### The expression analysis of the high affinity iron permease gene of R. oryzae (rFTR1)

*R. oryzae* 99-880 plugs taken from a confluent PDA plate were grown in potato dextrose broth overnight at 37°C with shaking. Mycelia were collected aseptically and transferred to fresh PDB flasks containing 350 µM ferric chloride (to suppress the expression of *rFTR1*), 350 µM ferric chloride plus 2 mM deferasirox to test iron chelation, or 2 mM deferasirox plus 6mM ferric chloride to supersaturate the added deferasirox. The flasks were incubated for 1 h at 37 °C with shaking. Mycelia were collected by filtration and ground in liquid nitrogen using mortar and pestle. Total RNA was extracted using RNeasy Plabt Mini™ kit (Qiagen®) with RLC buffer. The RNA was reverse-transcribed with oligo(dT) primer using the SuperScript™ First-Strand Synthesis System (Invitrogen®) to generate first-strand cDNA. The products were diluted and used in PCR to detect the expression of *FTR1* of *R. oryzae* and 18s rDNA gene. The sequences of the PCR primers are as follows: sense primer 5'-TCAGAGAAGGACTTGAAGC -3' and anti-sense primer 5'-TAAGTAGCCGTATTGTTCC -3' for *rFTR1* to amplify of *R. oryzae*; and sense primer 5'-CATGGTTGAGATTGTAAGATAG -3' and anti-sense primer 5'-AGTCAATGGACGTGGAGTC -3' for 18s rDNA gene. The PCR products were separated on a 2% agarose gel containing 0.1 µg/ml ethidium bromide. Both primer sets were designed to amplify a 400 bp bands.

### Green fluorescent protein (GFP) reporter assay

A 1.5 kb fragment upstream of the *rFTR1* ORF was PCR amplified using the following primer pair: sense primer 5'-GCTCTAGATCAGTCTCAACACCATCAATT-3'; and anti-sense primer 5'-TGCGGCCGTGCTTTTTAGTTCTCCTTGGA-3'. A 2.1 kb fragment containing the constitutively expressed actin promoter was also PCR amplified to use as a control using the following primer pair: sense 5'GCTCTAGATGGTATTATCGATTTAGA-3'; and anti-sense:5' TACGGCCGCATACCGGAACCGTTATCG-3. Amplified fragments were ligated into the *XbaI* and *EagI* sites of plasmid pyr225b (35). GFP was amplified from pGFPB21-43.31 (36), and cloned downstream of either promoter into *EagI-SacI* sites of the plasmid downstream of either promoter. Finally, a 2.1 kb fragment representing the ORF of *pyrF* and its native promoter was cloned into *SpeI-ClaI* sites of the plasmid to serve as a selection marker. Plasmids containing GFP driven by either rFTR1p or ACT1p were transformed into *R. oryzae* M16 with microprojectile particle bombardment (Biorad®). Transformants were selected on YNB+CSM-URA plates grown at 37°C for 5-7 days following bombardment. Isolates were then tested for uracil auxotrophy by passaging transformants on plates containing minimal medium without uracil and incubating the plates at 37°C. Purified transformants selected on uracil negative plates were analyzed by Southern blotting.

Expression of rFTR1p and ACT1p were studied in transformants grown in iron-replete medium (i.e. YNB+CSM-URA) or iron-depleted conditions (i.e. YNB+CSM-URA supplemented with 2mM deferasirox). Additionally, to reverse the effect of deferasirox transformants were grown in YNB+CSM-URA supplemented with 2mM deferasirox and supersaturating 6 mM ferric chloride. Finally, M16 transformed with empty plasmid (pyr225b-pyrF without GFP) was used as a negative control. Spores (1x 10⁴/ml) were inoculated in the above mentioned media and incubated overnight at 37°C. A drop of the culture was mounted on a slide and GFP expression was visualized in *R. oryzae* with a Leica DMRXE confocal microscope using an excitation wavelength of 488 nm. Additionally, a I ml sample from each culture was also used to quantify the fluorescence emission using a FACSCaliber™ (Becton Dickinson®) instrument equipped with an argon laser emitting at 488 nm. Spores were gated by forward and side scatter and fluorescence measured in FL1.

### Susceptibility testing

Minimum inhibitory concentration (MIC) and minimum fungicidal concentration (MFC) were determined for deferasirox by the method of Espinel-Ingroff using *R. oryzae* spores starved for iron (Espinel-Ingroff, (2001), *supra*). Cidality was defined as a ≤ 4-fold difference between MIC and MFC.

### Treatment regimens

Deferasirox (Novartis Pharmaceuticals, Basel, Switzerland) was suspended in 0.5% hydroxypropylcellulose (Klucel) and administered by oral gavage at 1, 3, or 10 mg/kg twice (qd) or every other day (qod). Treatment was begun 24 h post infection and continued for a total of 5 or 7 doses. Control groups were treated with the diluent, 5% dextrose water and 0.5% Klucel. In some experiments, a saturating dose of free iron was administered with deferasirox. Deferasirox is known to form molecular complexes with ferric iron (³⁺Fe) in a 1:2 ratio of iron to deferasirox. Based on the known molecular weights of ferric chloride (FeCl₃, molecular weight 162.22 g/mol) and deferasirox (molecular weight 373.4 g/mol), a 2.8mg/kg dose of FeCl₃ was calculated to result in a significant excess of ³⁺Fe vs. a 10 mg/kg dose of deferasirox given to an 18 g mouse: ³⁺Fe moles = (0.0028 g/kg * 0.018 kg [mouse weight]/162.22 glmol) = 3 x 10⁻⁷ mol vs. deferasirox moles = (0.01 g/kg * 0.018 kg [mouse weight]/ 373.4 g/mol) = 5 x 10⁻⁷ mol Ratio = 1:1.7 moles of ³⁺Fe to deferasirox, which is more than that required to achieve a saturating 1:2 ratio. LAmB diluted in 5% dextrose water was obtained from Gilead Sciences® (Foster city, CA) and was administered at a dose of 15 mg/kg daily (qd) via the tail vein for 4 days.

### Statistical analysis

The nonparametric log rank test was used to determine differences in survival times of the mice. Differences in tissue fungal burdens, splenic lymphocyte frequencies, and whole organ cytokines in the infected organs were compared by the Mann Whitney U test. P values of <0.05 were considered significant.

It has previously been shown (in Fu et al. FEMS Micorbiol. Lett. 235:169-176 (2004)) that that iron starvation causes the rapid expression of the high affinity iron permease gene (*rFTR1*) in *Rhizopus oryzae*, the most common cause of mucormycosis. Therefore, to confirm that deferasirox effectively chelates iron from *R. oryzae,* we defined the impact of *in vitro* deferasirox exposure on *R. oryzae* expression of *rFTR1. R. oryzae* 99-880 spores were incubated overnight in PDB. The next day mycelia were transferred into PDB supplemented with 350 µM ferric chloride (iron replete), 350 µM ferric chloride plus 2 mM deferasirox (iron depleted), or 2 mM deferasirox plus supersaturating 6 mM ferric chloride. RNA was extracted from spores grown in each condition for 1 h at 37°C, and RT-PCR was performed to evaluate gene expression. The *rFTR1* gene was expressed in the presence of deferasirox, but not in media supplemented with ferric chloride or in the presence of deferasirox plus supersaturating ferric chloride (Figure 8a).

To confirm rFTR1p expression and *rFTR1* promoter activity under chelating conditions, the gene encoding green fluorescent protein (GFP) was cloned behind the *rFTR1* promoter (Figure 8b). *R. oryzae* M16 spores transformed with the rFTR1::GFP construct or ACT1 p::GFP (constitutive positive control) were incubated overnight with deferasirox, deferasirox plus supersaturating ferric chloride, or iron-rich media (iron replete). Figure 8c shows that in contrast to GFP under the control of the *ACT1* promoter, which was constitutively expressed regardless of growth conditions, GFP under the control of the *rFTR* promoter was expressed or was active only in the presence of iron chelation conditions (deferasirox). As seen by confocal microscopy, M16 transformed with the constitutive ACT1 p::GFP construct was fluorescent regardless of growth conditions. In contrast, M16 transformed with rFTR1p::GFP was only fluorescent in the presence of deferasirox . Similarly, by flow cytometry, less than 1% of untransformed or rFTR1p::GFP-transformed spores grown in iron-replete conditions were fluorescent. In contrast, 43% of spores transformed with rFTR1p::GFP and grown in the presence deferasirox were fluorescent. Collectively, these data confirmed that deferasirox induced an iron-starvation response in *R. oryzae.*

We next determined the deferasirox susceptibilities of multiple clinical isolates of Mucorales (Table 3). The MIC₉₀s of deferasirox against *Mucor, non-oryzae Rhizopus spp.*, and *R. oryzae* were 3.12 to 6.25 µg/ml. MFCs were similar to MICs, and deferasirox was cidal for 28/29 (97%) of isolates. Of note, slight growth was seen within the first twelve hours even at extremely high concentrations of deferasirox. However the fungi were dead by 24 hours even at low concentrations of deferasirox, suggesting that deferasirox cidality was time-dependent, rather than concentration-dependent. Furthermore, the MICs and MFCs of deferasirox against these isolates were well below clinically achievable peak (∼38 µg/ml) and trough serum levels (∼17 µg/ml) of the drug at steady state when administered at the FDA-approved starting dose (20 mg/kg/d).

This example, in addition to demonstrating that deferasirox induced an iron-starvation response in *R. oryzae,* showed that deferasirox was cidal for multiple clinical isolates from two different genera of the family Mucorales.

**Table 3: Deferasirox MICs and MFCs Against Mucorales**

| | **24 h** | | | **48 h** | | |
|---|---|---|---|---|---|---|
| **Organism** | **MIC (µg/ml)** | **MFC (µg/ml)** | **Fungicidal?*** | **MIC (µg/ml)** | **MFC (µg/ml)** | **Fungicidal?*** |
| *Mucor* 97-1083 | 3.12 | 3.12 | Y | 3.12 | 3.12 | Y |
| *Mucor ramosissumus* 97-1192 | 0.78 | 0.78 | Y | 0.78 | 0.78 | Y |
| *Mucor sp.* 99-932 | 3.12 | 3.12 | Y | 3.12 | 3.12 | Y |
| ***Mucor spp.* MIC₉₀/MFC₉₀** | **3.12** | **3.12** | **Y** | **3.12** | **3.12** | **Y** |
| *R. microsporus* ATCC 62417 | 0.39 | 0.39 | Y | 0.39 | 0.39 | Y |
| *Rhizopus* sp. 99-1150 | 6.25 | 6.25 | Y | 3.12 | 3.12 | Y |
| *Rhizopus* sp. 99-1700 | 6.25 | 6.25 | Y | 1.56 | 1.56 | Y |
| *Rhizopus* sp. ATCC 20577 | 3.12 | 6.25 | Y | 3.12 | 6.25 | Y |
| ***Non-oryzae Rhizopus* MIC₉₀/MFC₉₀** | **6.25** | **6.25** | **Y** | **3.12** | **6.25** | **Y** |
| *R. oryzae* 729-02 | 12.5 | 12.5 | Y | 6.25 | 6.25 | Y |
| *R. oryzae* 99-133 | 6.25 | 6.25 | Y | 1.56 | 6.25 | Y |
| *R. oryzae* 99-880 | 3.12 | 3.12 | Y | 3.12 | 3.12 | Y |
| *R. oryzae* 99-892 | 1.56 | 1.56 | Y | 1.56 | 1.56 | Y |
| *R. oryzae* HUMC 02 | 12.5 | 12.5 | Y | 1.56 | 1.56 | Y |
| *R. oryzae pyr17 pyrG-*/*-* | 0.78 | 0.78 | Y | 0.78 | 0.78 | Y |
| *R. oryzae* type I NRRL 10206 | 3.12 | 3.12 | Y | 3.12 | 3.12 | Y |
| *R. oryzae* type I NRRL 21251 | 3.12 | 6.25 | Y | 6.25 | 6.25 | Y |
| *R. oryzae* type I NRRL 6059 | 6.15 | 6.25 | Y | 1.56 | 6.25 | Y |
| *R. oryzae* type I NRRL 11823 | 3.12 | 3.12 | Y | 6.25 | 6.25 | Y |
| *R. oryzae* type I NRRL13142 | 6.25 | 6.25 | Y | 3.12 | 3.12 | Y |
| *R. oryzae* type I NRRL13440 | 1.56 | 12.5 | N | 1.56 | 12.5 | N |
| *R. oryzae* type I NRRL13533 | 1.56 | 6.25 | Y | 1.56 | 6.25 | Y |
| *R. oryzae* type I NRRL21396 | 12.5 | 12.5 | Y | 1.56 | 6.25 | Y |
| *R. oryzae* type I NRRL21409 | 12.5 | 1.56 | Y | 1:56 | 1.56 | Y |
| *R. oryzae* type II NRRL 18148 | 0.78 | 0.78 | Y | 0.78 | 0.78 | Y |
| *R. oryzae* type II NRRL 21446 | 6.25 | 12.5 | Y | 6.25 | 6.25 | Y |
| *R. oryzae* type II NRRL 21447 | 6.25 | 12.5 | Y | 6.25 | 6.25 | Y |
| *R. oryzae* type II NRRL 21477 | 1.56 | 1.56 | Y | 1.56 | 1.56 | Y |
| *R. oryzae* type II NRRL 21628 | 0.78 | 0.78 | Y | 0.78 | 0.78 | Y |
| *R. oryzae* type II NRRL 21789 | 0.39 | 0.39 | Y | 0.39 | 0.39 | Y |
| *R. orzae type* II 10206 | 3.12 | 3.12 | Y | 3.12 | 3.12 | Y |
| ***R. oryzae* MIC₉₀IMFC₉₀** | **12.5** | **12.5** | **Y** | **6.25** | **6.25** | **Y** |
| *Cidal = MFC/MIC ≤ 4(37) | | | | | | |

### EXAMPLE V

### The Efficacy of Deferasirox against R. Oryzae in vivo

This Example shows the *in vivo* efficacy of deferasirox in treating mice infected with Mucorales.

The following describes materials and methods used in the procedures described in this or the subsequent examples.

### Murine Models

For *in vivo* infection, BALB/c male mice (>20g) were rendered diabetic with a single i.p. injection of 210 mg/kg streptozotocin in 0.2 ml citrate buffer 10 days prior to fungal challenge. Glycosuria and ketonuria were confirmed in all mice 7 days after streptozotocin treatment. For neutropenic mouse model, mice were injected with a single i.p. dose of200 mg/kg cycolphosphamide (Bristol Myer Squibb) 2 days prior to infection with *R. oryzae.* This treatment regimen resulted in pancytopenia from days -2 to day 5 relative to infection, with recovery of cell counts began on day 6 post infection. Mice were infected through the tail vein with the appropriate inocula of *R. oryzae.* To confirm the inocula, dilutions were streaked on PDA plates containing 0.1% triton and colonies were counted following a 24 h incubation period at 37°C. The primary efficacy endpoint was time to death. As a secondary endpoint, brain fungal burden (the primary target organ) was determined by homogenization by rolling a pipette on organs placed in Whirl-Pak™ bags (Nasco®, Fort Atkinson, WI) containing 1 ml saline. The homogenate was serially diluted in 0.85% saline and then quantitatively cultured on PDA. Values were expressed as log₁₀ cfu g₋₁ tissue. Finally, for histopathological analysis, infected organs were collected from mice and fixed in 10% zinc formal in. Fixed tissues were embedded in paraffin, and 5 mm sections were stained with haematoxylin and eosin for light microscopy examination. All procedures involving mice were approved by the institutional animal use and care committee, following the National Institutes of Health guidelines for animal housing and care.

To determine if deferasirox's *in vitro* activity would translate into *in vivo* efficacy, we utilized our murine model of disseminated mucormycosis in diabetic ketoacidotic mice. Diabetic ketoacidotic mice were infected via the tail-vein with 2.2 x 10⁴ spores of *R. oryzae* 99-892. The mice were treated by oral gavage with 1, 3, or 10 mg/kg deferasirox in 0.5% hydroxypropylcellulose (Klucel) twice daily (bid) for seven days starting the day after infection. Negative control mice were treated with hydroxypropylcellulose carrier (placebo) or deferasirox plus saturating ferric chloride (administered subcutaneously). An additional negative control consisted of uninfected mice treated with ferric chloride. Deferasirox at 1, 3, or 10 mg/kg bid significantly improved survival compared to controls (Figure 9a).

In a separate experiment, deferasirox was found to be effective against a second clinical isolate of *R. oryzae,* the 99-880 isolate. Diabetic ketoacidotic mice were infected via the tail-vein with 1.3 x 10³ spores of the more virulent *R. oryzae* 99-880 and treated as above with deferasirox at 10 mg/kg bid or placebo for seven days. Deferasirox significantly improved time to death of mice infected with *R. oryzae* compared to placebo (Figure 9b).

To determine the impact of deferasirox on tissue fungal burden, diabetic ketoacidotic mice were infected via the tail-vein with 4.2 x 10⁴ spores of *R. oryzae* 99-892. Mice were treated with deferasirox (10 mg/kg bid), deferasirox plus saturating ferric chloride, or placebo. Treatment was begun 16 h after infection and administered daily for 3 days. Kidneys and brains were removed on day four, homogenized, and quantitatively cultured. Deferasirox resulted in a greater than 10-fold decrease in both brain and kidney (primary target organs) fungal burden compared to mice treated with placebo or deferasirox plus saturating ferric chloride (Figure 10a). By histopathology, kidneys of deferasirox-treated mice had no visible hyphae (as pointed to by arrows in Figure 10b), whereas kidneys of mice treated with placebo or deferasirox plus saturating ferric chloride had extensively filamented fungi. Furthermore, mice treated with saturating iron had a striking absence of neutrophil influx to the sites of infection, while neutrophil influx was prominent in the kidneys of mice treated with deferasirox (Figure 10b).

In addition to showing the direct antifungal effects of deferasirox, this example demonstrates that the protective effect of deferasirox can be reversed by administration of free iron, which confirms that the drug's mechanism of protection is via iron chelation.

### EXAMPLE VI

### Effects of Iron Chelation and Excess Iron on Host Immune Response

This Example shows a comparison between the effects of deferasirox and deferasirox plus ferric chloride on host immune response.

The following describes materials and methods used in the procedures described in this or the subsequent examples.

### Splenic Lymphocyte Frequency and Whole Organ Cytokines Assays

Splenic lymphocyte frequencies were determined as we have previously described (Spellberg et al., Infect. Immun. 71:5756-5764 (2003)). In brief, splenic homogenates were passed through 70 µm filters followed by RBC lysis with 0.15 M ammonium chloride. The cells were fixed with Cytofix™ buffer (BD Pharmingen®), permeabilized with Cytoperm™ buffer (BD Pharmingen), and stained with 10 µg/ml of FITC- conjugated anti-mouse CD4 (clone RM4-5), PE-conjugated anti-mouse IFN-γ (clone XMG1.2) or isotype control (clone R-34), allophycocyanin (APC)-conjugated anti-mouse IL-4 (clone 11B11) or isotype control (clone R3-34), or APC-conjugated anti-mouse IL-10 (clone JES5-16E3) or isotype control (clone A95-1) (all from BD Pharmingen). In separate experiments, the frequency of CD4+CD25+foxp3+ T-regutatory cells were determined using the Mouse Regulatory T cell Staining Kit (eBioscience®) per the manufacturer's recommendation. The frequency of apoptosis was determined using the Annexin FITC Apoptosis™ kit (BD Pharmingen®).

Cells were washed and three-color flow cytometry was performed on a Becton-Dickinson FACScan™ instrument calibrated with CaliBRITE™ beads (BD Pharmingen) using FACSComp™ software as per the manufacturer's recommendations. During data acquisition, CD4+ lymphocytes were gated by concatenation of forward and side scatter, and FITC-anti-CD4 antibody fluorescence properties. Data for each sample were acquired until 10,000 CD4+ lymphocytes were analyzed.

### Whole Organ Cytokines Assay

Spleens and kidneys were homogenized in 1 ml of PBS. The homogenates were pelleted at maximum speed on a tabletop centrifuge at 4°C. The supernatants were assayed for cytokines using the Cytometric Bead Array Murine Inflammatory Cytokine™ kit (BD Pharmingen) per the manufacturer's instructions.

Because of the alterations in inflammatory cellular influx seen in kidneys of mice treated with saturating ferric chloride vs. iron chelation, in this example we determined the impact of deferasirox therapy on Th1/Th2 and inflammatory cytokine responses in infected mice. Diabetic ketoacidotic mice were infected with 3.1 x 10⁴ spores of *R. oryzae* 99-892 as above and treated with deferasirox, deferasirox plus ferric chloride, or placebo. On day four of infection, spleens and kidneys were processed for intracellular and whole organ cytokine determination. Deferasirox resulted in a significant increase in both Th1 and Th2 splenocyte frequencies compared to mice treated with saturating ferric chloride or placebo (Figure 11a). The frequencies of CD4+IL-10+ or CD4+CD25+foxp3+ splenocytes were not significantly different between the groups (data not shown). There was also no significant difference in splenocyte apoptosis between the groups (data not shown).

Deferasirox-treated mice had significantly higher splenic levels of the canonical pro-inflammatory cytokines, TNF and IFN-γ, than mice treated with saturating iron or placebo (Figure 11 b). Mice treated with deferasirox also had significantly higher kidney levels of IFN-γ (Figure 11b).

This study shows that deferasirox non-specifically stimulated the suppressed inflammatory response in diabetic ketoacidotic mice, increasing the frequency of both Th1 and Th2 lymphocytes, as well as splenic and kidney inflammatory cytokine levels. Furthermore, reversal of chelation by administration of a supersaturing iron dose decreased the number of neutrophils seen in infected kidneys.

### EXAMPLE VII

### Deferasirox (Exjade^{®}) Used in Combination with Liposomal Amphotericin B for the Therapeutic Treatment of Murine Mucormycosis

This Example shows the effects of combination therapy using the iron chelator, Deferasirox (Exjade^{®}), and Liposomal Amphotericin B (LAmB) for the treatment of mucormycosis.

As described previously, clinical and animal model data indicate that the presence of elevated available serum iron predisposes the host to mucormycosis. Exemplified in the above Examples, Deferasirox iron chelation therapy was demonstrated to improve the survival of diabetic, ketoacidotic mice infected with *R. oryzae,* the most common cause of mucormycosis. To corroborate Deferasirox's effectiveness in adjunctive therapy, the efficacy of Deferasirox combined with LAmB was compared to either drug alone for the treatment of the animal model murine mucormycosis.

Methods, animal procedures and reagents were performed and/or prepared as described previously. Briefly, BALB/c mice with streptozotocin-induced DKA were infected via the tail-vein with 2.0 x 10³ spores of *R. oryzae.* Treatment was initiated 24 h post infection with: (1) LAmB (15 mg/kg iv) given once daily for 4 doses; (2) Deferasirox (10 mg/kg po) given twice daily for 7 doses, or (3) both LAmB and Deferasirox using the above doses. Placebo mice received vehicle control. Endpoints were time to death and colony forming units (CFU).

The results of the above study are shown in Figures 12 and 13. Figure 12 shows the efficacy for the monotherapies Deferasirox alone and LAmB alone as well as for the combination therapy Deferasirox and LAmB together in treating mucormycosis. The results indicate that both monotherapies improved survival compared to placebo (25% and 28% survival for Deferasirox and LAmB, respectively, and 0% for placebo, P<0.003). Combination therapy markedly improved both time to survival and overall survival of infected DKA mice (n > 16 per group) compared to all other groups (70% survival for combination therapy, P<0.008 by Log Rank test for all comparisons).

The results of these treatments with respect to target organs is shown in Figure 13. The results indicate that neither drug alone was able to reduce brain CFUs (primary target organ) compared to placebo. However, combination therapy with both Deferasirox and LAmB reduced brain CFUs by more than 2 logs compared to all other arms (*P* <0.04). Only LAmB and combination therapy were able to reduce kidney CFU (secondary target organ) compared to placebo-treated mice (*P*<0.01).

This study indicates that iron chelation therapy and high dose LAmB can be equally effective in treating experimental mucormycosis in DKA. Combination therapy with LAmB and Deferasirox was more effective than either drug alone. These data further corroborate the therapeutic efficacy of using iron chelation such as Deferasirox as an adjunctive therapy with a lipid formulation of amphotericin B in treating mucormycosis infections. The above results further indicate that, when used in combination, the iron chelator Deferasirox (Exjade^{®}) and Liposomal Amphotericin B result in a synergistic effect for the treatment of mucormycosis in animal models.

### EXAMPLE VIII

### Efficacy of Deferasirox in Neutropenic, Infected Mice

This Example shows the efficacy of deferasirox in treating neutropenic mice infected with *R. oryzae.*

To determine if deferasirox was also effective in the setting of neutropenia, mice were myeloablated with cyclophosphamide. Two days later, mice were infected via the tail-vein with 2.7 x 10³ spores of *R. oryzae* 99-892. Initial dose response studies suggested that, in contrast to the diabetic ketoacidotic model, optimal outcomes were achieved with dosing of deferasirox every other day rather than every day (data not shown), as we have previously described with the iron chelator, deferiprone. Treatment with deferasirox (10 mg/kg twice qod for five doses starting 24 h post infection) significantly improved time to death compared to placebo (Figure 14). In contrast, deferasirox dosed twice daily did not significantly improve time to death versus placebo.

This study indicates that the Maximal efficacy of deferasirox in neutropenic mice required less frequent dosing than in the diabetic ketoacidotic mouse model, and deferasirox appeared to be somewhat more effective in diabetic ketocacidotic mice than in neutropenic mice.

### EXAMPLE IX

### Evaluation of Specific Toxicity of Deferasirox in Neutropenic Mice

This Example shows the results of toxicity evaluation in neutropenic mice treated with daily dose of deferasirox.

The following describes materials and methods used in the procedures described in this example.

### Deferasirox toxicity studies

Toxicity of deferasirox in neutropenic mice was evaluated. Mice were rendered neutropenic as above and treated with deferasirox at 10 mg/kg twice every day or every other day for 7 days. Mice from three different groups (i.e. placebo, deferasirox daily treatment, and deferasirox every other day treatment) were sacrificed on day 3 or 8 and blood was collected and sent for Charles River Laboratories for evaluation. Additionally, bone marrow smears were prepared from femurs, and tissues were collected, preserved in zinc-buffered formalin, embedded in paraffin, sectioned at 5µm, and stained with hematoxylin and eosin. The resulting slides were examined by a board-certified veterinary pathologist at Charles River Laboratories.

Because deferasirox dosing every other day was optimal in the neutropenic model, we sought to identify any potential toxicity caused by daily dosing of deferasirox in neutropenic mice. Mice were made neutropenic with cyclophosphamide as above, but were not infected. The mice were treated with deferasirox 10 mg/kg twice daily for seven days, 10 mg/kg twice every other day for 4 doses, or placebo. Terminal bleeds were performed on day 3 or day 8 to measure complete blood count, serum chemistries, and liver function tests. Histopathology was performed on an extensive list of organs, including brain, heart, lungs, liver, gallbladder, spleen, kidneys, gastrointestinal tract (including stomach, small intestine, and large intestine), and bone marrow (both smears and core). No differences in white cell counts, absolute neutrophil counts, platelet counts, hemoglobin levels, serum chemistries (including creatinine, blood urea nitrogen, or electrolytes), or liver function tests (including AST, ALT, or bilirubin) were identified between the three groups at either time point. By histopathology, no organ-specific toxicity attributable to deferasirox was identified, including no evidence of alterations in hematopoeisis. Specifically, there was no evidence of exacerbation of or delayed recovery from bone marrow ablation by chemotherapy, nor was there any evidence of renal or hepatic dysfunction by laboratory testing, nor any specific organ toxicity by histopathological evaluation.

This study shows that the diminished activity of daily deferasirox dosing in neutropenic mice could not be due to a toxic effect of deferasirox in such mice since no evidence of toxicity was found.

Throughout this application various publications have been referenced within parentheses. The disclosures of these publications in their entireties are hereby incorporated by reference in this application in order to more fully describe the state of the art to which this invention pertains.

Although the invention has been described with reference to the disclosed embodiments, those skilled in the art will readily appreciate that the specific examples and studies detailed above are only illustrative of the invention. It should be understood that various modifications can be made without departing from the spirit of the invention. Accordingly, the invention is limited only by the following claims.

## Claims

1. An iron chelating compound for use in a method of treating or preventing a fungal condition, comprising administering to an individual having, or susceptible to having, a fungal condition a therapeutically effective amount of at least one iron chelating compound for a sufficient time to reduce the severity of a fungal condition, wherein said iron chelating compound comprises a non-siderophore or non-xenosiderophore relative to said fungal condition.

2. The iron chelating compound for use according to claim 1 , wherein said fungal condition comprises zygomycosis, aspergillosis, cryptococcosis, candidiasis, histoplasmosis, coccidiomycosis, paracoccidiomycosis, fusariosis (hyalohyphomycoses), blastomycosis, penicilliosis or sporotrichosis.

3. The iron chelating compound for use according to claim 2, wherein said zygomycosis further comprises mucormycosis.

4. The iron chelating compound for use according to claim 3, wherein said mucormycosis comprises rinocerebral mucormycosis, pulmonary mucormycosis, gastrointestinal mucormycosis, disseminated mucormycosis, bone mucormycosis, mediastinum mucormycosis, trachea mucormycosis, kidney mucormycosis, peritoneum mucormycosis, superior vena cava mucormycosis or external otitis mucormycosis.

5. The iron chelating compound for use according to claim 4, wherein said mucormycosis is associated with an infectious agent within the order Mucorales.

6. The iron chelating compound for use according to claim 5, wherein said agent within the order Mucorales is selected from a *Rhizopus* species consisting of *Rhizopus oryzae (Rhizopus arrhizus), Rhizopus microsporus* var. *rhizopodiformis, Absidia corymbifera, Apophysomyces elegans, Mucor* species, *Rhizomucor pusillus* and *Cunninghamella* spp (*Cunninghamellaceae* family).

7. The iron chelating compound for use according to claim 2, wherein said candidiasis is associated with an infectious agent selected from a *Candida* species consisting of *Candida albicans, Candida krusei, Candida tropicalis, Candida glabrata* and *Candida parapsilosis.*

8. The iron chelating compound for use according to claim 2, wherein said aspergillosis is associated with an infectious agent selected from an *Aspergillus* species consisting of *Aspergillus fumigatus, Aspergillus flavus, Aspergillus terreus, Aspergillus nidulans* and *Aspergillus clavatus.*

9. The iron chelating compound for use according to claim 1, wherein said iron chelating compound comprises deferiprone or deferasirox.

10. The iron chelating compound for use according to claim 1, further comprising two or more iron chelating compounds, wherein each iron chelating compound comprises a non-siderophore or non-xenosiderophore relative to said fungal condition.

11. The iron chelating compound for use according to claim 10, wherein said two or more iron chelating compounds comprise deferiprone and deferasirox.

12. The iron chelating compound for use according to claim 1, wherein said preventing comprises prophylactic administration of said at least one iron chelating compound prior to onset of said fungal condition.
